# EUROPEAN PATENT APPLICATION

(11) **EP 4 043 884 A1**
(43) Date of publication of application: **17.08.2022**
(21) Application number: 21156314.3
(22) Date of filing: 10.02.2021
(51) Int. Cl.: G01N 33/92

(54) **MEDIUM-CHAIN FATTY ACYLS AND PHOSPHOLIPIDS AS BIOMARKERS FOR CARDIOVASCULAR DISEASES**

(71) Applicant: Eberhard-Karls-Universität Tübingen, 72074 Tübingen (DE)
(72) Inventor: LÄMMERHOFER, Michael, 72076 Tübingen (DE); GAWAZ, Meinrad, 72076 Tübingen (DE); DITTRICH, Kristina, 72076 Tübingen (DE)
(74) Representative: Kuttenkeuler, David

(57) **Abstract**

The present invention relates to the use of lipid compounds comprising at least one medium chain fatty acid as biomarkers for diseases which are due to or accompanied by fatty acid oxidation disorder. The present invention furthermore relates to methods of assessing the risk to develop said diseases by determining the lipid compounds in samples.

## Description

The present invention relates to the use of lipid compounds comprising at least one medium chain fatty acid as biomarkers for diseases which are due to or accompanied by fatty acid oxidation disorder. The present invention furthermore relates to methods of assessing the risk to develop said diseases by determining the lipid compounds in samples.

### BACKGROUND OF THE INVENTION

Cardiovascular diseases (CVDs) are according to WHO the number one cause of death globally. CVDs are a group of disorders of the heart and blood vessels including coronary heart disease and cerebrovascular disease. Major risk factors are elevated blood pressure, hyperlipidemia, diabetes, overweight and obesity.

In routine clinical diagnostics, total cholesterol, LDL and HDL cholesterol, as well as triglycerides are typically determined as risk factors nowadays. However, the predictive quality of those risk markers is limited. Therefore, identification of patients at higher risk and measurement of additional risk markers, e.g. high-sensitivity C-reactive protein (hsCRP), lipoprotein (a) (Lp[a]) or ratios of certain ceramides, in individuals who are at intermediate risk (e.g. subjects with atherosclerosis, strong family history) for developing cardiovascular disease has been recommended. Early detection allows proper health management ranging from changes of life style to proper medication, such as antiplatelet therapy or lipid lowering drugs. Considering the adverse effects of hyperlipidemia, patients with significant risk of heart disease are nowadays often treated with statins which have shown beneficial therapeutic effects.

The vulnerability of humans for risk to develop CVD is nowadays commonly assessed by clinical markers which are related to lipid metabolism. As mentioned, the predictive quality is limited and hence there is a strong quest for new biomarkers with better predictive and prognostic properties. The important role of lipids in platelet activity was highlighted in a recent study on platelet phosphoACC which was found to be a risk marker in patients with suspected CAD and provided additional evidence of an interplay between platelets and lipids in atherosclerosis (Kautbally *et al.*, 2019). A clear relationship between platelet phosphoACC and CAC Agatston score severity, and acute coronary events in CAD was demonstrated.

It is, thus, an objective of the present invention to provide improved means and methods for assessing the risk of patients to develop CVD and similar diseases.

### SUMMARY OF THE INVENTION

According to the present invention this object is solved by using a lipid compound comprising a medium chain fatty acid of general formula 1 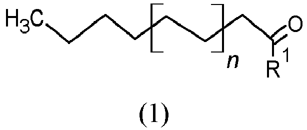 wherein
n is 0, 1, 2 or 3, preferably 1 or 2,
   and
R₁ is -OH or a lipid backbone of a lipid selected from fatty acyls, glycerolipids, glycerophospholipids, sphingolipids, and sterol lipids,
wherein said MCFA is optionally substituted and/or optionally unsaturated,
as biomarker for assessing the risk to develop a disease which is due to or accompanied by fatty acid oxidation disorder, said risk assessment preferably comprising diagnosis, prognosis, prediction and/or monitoring of said disease.

According to the present invention this object is solved by a method for assessing the risk to develop a disease due to or accompanied by fatty acid oxidation disorder, said method comprising
(a) providing a sample obtained from a human,
(b) determining the relative or absolute concentration of at least one lipid compound in said sample, wherein said lipid compound is as defined herein.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 20" should be interpreted to include not only the explicitly recited values of 1 to 20, but also include individual values and sub-ranges within the indicated range. Thus, included in this numerical range are individual values such as 1, 2, 3, 4, 5 .... 17, 18, 19, 20 and sub-ranges such as from 2 to 10, 8 to 15, etc. This same principle applies to ranges reciting only one numerical value, such as "at least 2". Furthermore, such an interpretation should apply regardless of the breadth of the range or the characteristics being described.

### MCFA-containing lipids as biomarkers for CVDs

As outlined above, the present invention provides the use of a lipid compound comprising at least one medium chain fatty acid (MCFA) of general formula 1 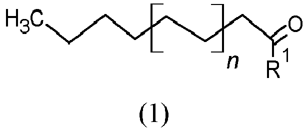 wherein
n is 0, 1, 2 or 3, preferably 1 or 2,
   and
R₁ is -OH or a lipid backbone,
wherein said MCFA is optionally substituted and/or optionally unsaturated,
as biomarker for assessing the risk to develop a disease which is due to or accompanied by fatty acid oxidation disorder.

Said risk assessment preferably comprises diagnosing, prognosting, predicting and/or monitoring of said disease.

Said lipid backbone is a lipid backbone from a lipid of the following lipid categories as defined by the lipid maps consortium (https://www.lipidmaps.org; Fahy *et al.,* 2009):
- fatty acyls (FA),
- glycerolipids (GL),
- glycerophospholipids (GPL),
- sphingolipids (SPL), and
- sterol lipids (ST).

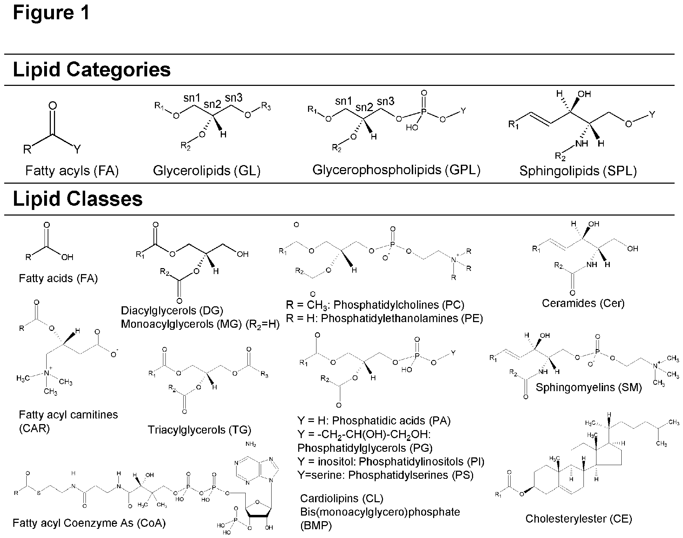

Said lipid categories are also shown in Figure 1 along with their major lipid (sub)classes.

A "medium chain fatty acid" (MCFA) as used herein refers to a C6 to C12 fatty acid, such as C6 (hexanoic acid), C8 (octanoic acid), C10 (decanoic acid), C12 (dodecanoic acid).

The medium chain fatty acids (MCFA) of the lipid compounds of the present invention are optionally substituted or optionally unsaturated (with double bond).
For example, 3-hydroxy or 3-oxo fatty acyls, which are indermediates of the fatty acid oxidation cycle.
For example, 2,3-enoyl-fatty acyls, which are indermediates of the fatty acid oxidation cycle. For example, C10:1 fatty acyl, such as C10:1-fatty acid, FA 10:1.

Throughout the specification, when there is reference made to a medium chain fatty acid (MCFA) this always comprises an optionally substituted or unsaturated MCFA.

In contrast thereto,
- "short chain fatty acids" (SCFAs) are C2 to C4 fatty acids, such as C2 (acetic acid), C4 (butyric acid);
- "long chain fatty acids" (LCFAs) are C14 to C18 fatty acids, such as C14 (myristic acid), C16 (palmitic acid), C18 (stearic acid);
- "very long chain fatty acids" (VLCFAs) are C20 to C28 fatty acids, such as C20 (arachidic acid) and C22 (docosanoic acid);
- "ultra-long chain fatty acids" (ULCFAs) are C30 to C38 fatty acids.

When they are connected to a lipid backbone or other residue(s), their corresponding fatty acyl residues (lipid side chains) are termed likewise, i.e. SC-, MC-, LC-, VLC-, ULC-fatty acyls.

Herein, we use shorthand notations of lipids as widely accepted in the scientific community. The following rules and definitions are applied throughout this text:
- The lipid is primarily specified by the abbreviation of the lipid class, e.g. PC for phosphatidylcholine.
- The abbreviation of the lipid class is followed by the number of fatty acyl carbons and double bonds (separated by a colon), e.g. PC 18:0 corresponding to a phosphatidylcholine with fatty acids in sn1 and sn2 positions (sn, stereospecific numbering; refers to the position at the glycerol backbone) having in total 18 carbon atoms and 0 double bonds. This corresponds to the sum composition and is used when the fatty acid side chains are not known.
- If the side chains are known, they are specified separately, the fatty acid (FA) at sn1 position first followed by the fatty acid at sn2 separated by a slash, e.g. as PC 10:0/8:0 or PC 10:0-8:0. In the former, indicated by a slash (/), the decanoic acid (FA 10:0) is attached at sn1 position and octanoic acid (FA 8:0) at sn2. In the latter case, indicated by a hyphen (-), the fatty acids are known but their position at sn1 or sn2 is unknown. Hence, PC 10:0-8:0 comprises two possibilities: it can be either PC 10:0/8:0 or PC 8:0/10:0. All of these lipids have the same sum composition of PC 18:0.
- Ether lipids, having instead of the fatty acyl group either an alkyl or alkenyl substituent at sn1, are indicated by an e suffix subsequent to the lipid shorthand notation, e.g. PC32:1e means this PC has an ether group at sn1 instead of an acyl moiety. It is tantamount to PC(O-16:0/16:1) (the O prefix is used to indicate the alkyl ether subsitutent) or PC(P-16:0/16:0) (here the double bond is already considered by the P prefix). PC32:1e comprises both options.
- If the specified lipid shorthand notation allows several stereoisomers and constitutional isomers, the specification comprises all of them.

In a preferred embodiment, in the lipid compound R₁ is -OH or a lipid backbone of fatty acyls (FA), glycerolipids (GL), or glycerophospholipids (GP).

### - Fatty acyls (FA)

In one embodiment, the lipid compound is a free fatty acid with R₁ being -OH,
wherein preferably n = 1 (C8:0-fatty acid, FA 8:0) or n = 2 (C10:0-fatty acid, FA 10:0).

C8:0-fatty acid (FA 8:0):

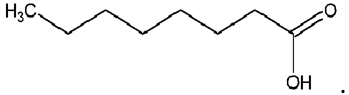

C10:0-fatty acid (FA 10:0):

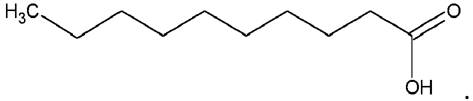

For example, n = 2 with one double bond (C10:1-fatty acid, FA 10:1).

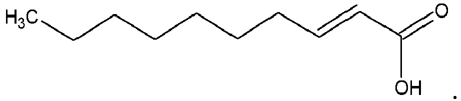

In one embodiment, the lipid compound is selected from fatty acyl coenymes A (CoAs) with R₁ being -SCoA, of formula 2: 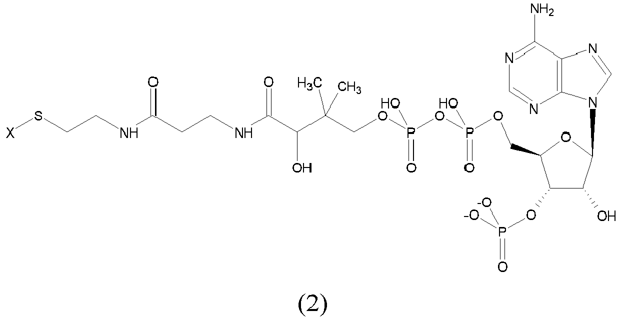 wherein X is a medium chain fatty acid (MCFA).

Preferably, n is 1 (C8:0-acyl-coenzyme A) or n is 2 (C10:0-acyl-coenzyme A).
C8:0-acyl-coenzyme A (CoA 8:0): 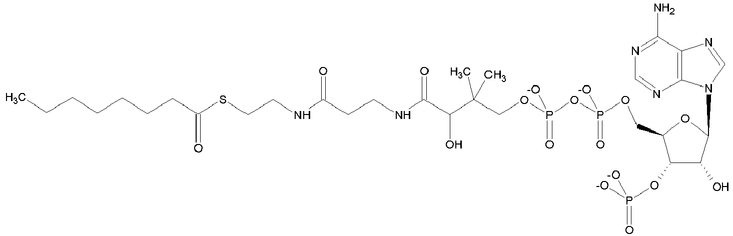
C10:0-acyl-coenzyme A (CoA 10:0):

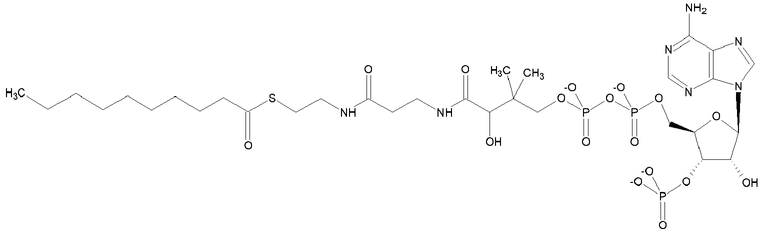

In one embodiment, the lipid compound is selected from acylcarnitines (CAR) with R₁ being carnitine, of formula 3: 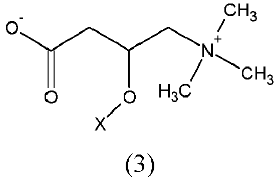 wherein X is a medium chain fatty acid (MCFA).

Preferably, n is 1 (C8:0-acylcarnitine, CAR 8:0) or n is 2 (C10:0-acyl-carnitine, CAR 10:0).
C8:0 acylcarnitine (CAR 8:0): 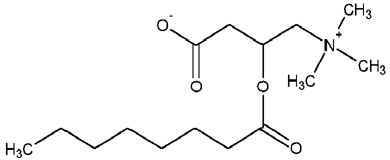
C10:0-acyl-carnitine (CAR 10:0):

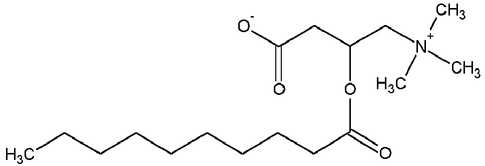

### - Glycerophospholipids (GPL)

In one embodiment, the lipid compound is selected from phosphatidylcholines (PC) of formula 4: 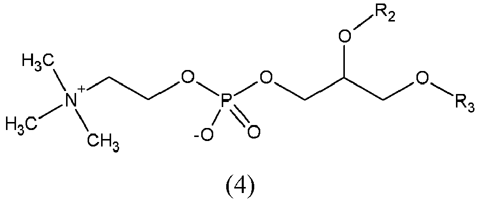 wherein R₂ and R₃ are fatty acids with at least one of R₂ and R₃ being a medium chain fatty acid (MCFA), wherein in said MCFA n is preferably 1 or 2 (FA8:0 or FA10:0)

Preferably, a lipid compound being a phosphatidylcholine of formula 4 is selected from
PC(8:0/8:0),
PC(10:0/8:0),
PC(8:0/10:0),
PC(10:0/10:0),
PC(16:0/8:0),
PC(8:0/16:0),
PC(16:0/10:0),
PC(10:0/16:0),
PC(10:0/20:4) or
PC(20:4/10:0).

For example, PC(10:0/10:0):
(sum composition also named as PC(20:0) herein)
1,2-Didecanoyl-sn-glycero-3-phosphocholine
CAS Registry Number 3436-44-0
C₂₈ H₅₆ N O₈ P
3,5,9-Trioxa-4-phosphanonadecan-1-aminium, 4-hydroxy-*N,N,N*-trimethyl-10-oxo-7-[(1-oxodecyl)oxy]-, inner salt, 4-oxide, (7*R*) 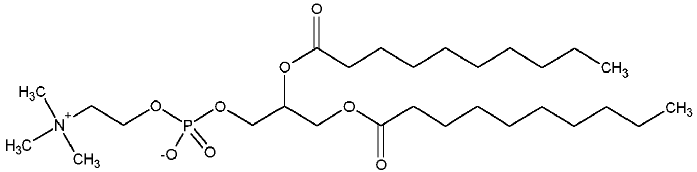
PC(10:0/8:0):
   (sum composition also named as PC(18:0) herein)
   1-Decanoyl-2-octanoyl-sn-glycero-3-phosphocholine 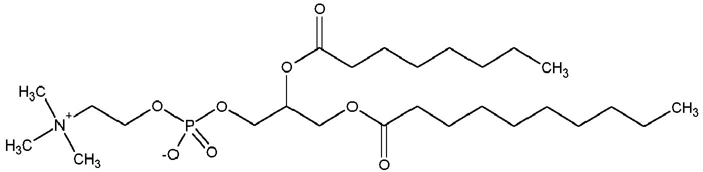
PC(8:0/10:0):
   (sum composition also named as PC(18:0) herein)
   1-Octanoyl-2-decanoyl--sn-glycero-3-phosphocholine 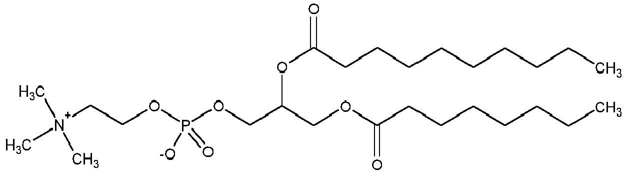
PC(16:0/8:0)
   (sum composition also named as PC(24:0) herein)
   1-Hexadecanoyl-2-octanoyl-sn-glycero-3-phosphocholine
PC(10:0/20:4)
   (sum composition also named as PC(30:4) herein)
   1-Decanoyl-2-arachidonoyl-sn-glycero-3-phosphocholine

In one embodiment, the lipid compound is selected from compounds of formula 5: 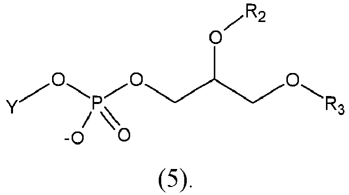 wherein
Y is H (PA, phosphatidic acids), or is a head group selected from the group comprising choline (PC, phosphatidylcholines),
ethanolamine, i.e. NH₂-CH₂-CH₂- (PE, phosphatidylethanolamines),
inositol (PI, phosphatidylinositols),
glycerol (PG, phosphatidylglycerols), or
serine (PS, phosphatidylserines).

In said compounds of formula 5, R₂ and R₃ are H or fatty acyl(s), with at least one of R₂ and R₃ being a MCFA, wherein in said MCFA n is preferably 1 or 2.

### - Glycerolipids (GL)

In one embodiment, the lipid compound is selected from compounds of formula 6:

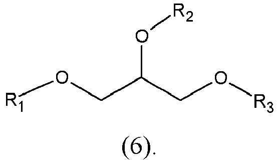

In one embodiment, R₁ is H:

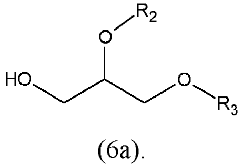

In one embodiment of compounds of formula 6a, R₂ and R₃ are both fatty acyls (DG, diacylglycerols) with at least one of R₂ and R₃ being a MCFA, wherein in said MCFA n is preferably 1 or 2.

For example, DG(8:0-10:0).

In one embodiment of compounds of formula 6a, one of R₂ and R₃ is H (MG, monoacylglycerols) and the other one is a MCFA, wherein in said MCFA n is preferably 1 or 2.

MGs can be the precursors of DG compounds.

In one embodiment of compounds of formula 6, R₁, R₂ and R₃ are fatty acyls (TG, triacylglycerols) with at least one of R₁, R₂ and R₃ being a MCFA, wherein in said MCFA n is preferably 1 or 2.

### - Sphingolipids (SPL)

In one embodiment, the lipid compound is selected from compounds of formula 7 or 8 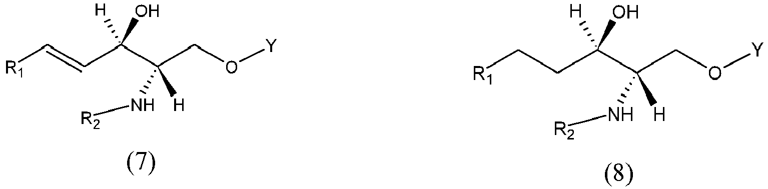 wherein
Y is selected from
H (ceramides),
hexosyl (glucosyl or galactosyl (HexCer) and sulfated forms),
dihexosyl (lactosyl-ceramides and sulfated forms), or
phosphocholine (sphingomyelines (SMs).

In one embodiment of compounds of formula 7 or 8:
R₁ is C9-C15 alkyl, and
R₂ is a fatty acyl with a MCFA, wherein in said MCFA n is preferably 1 or 2.

### - further glycerophospholipids (GPL)

In one embodiment, the lipid compound is selected from compounds of formula 9 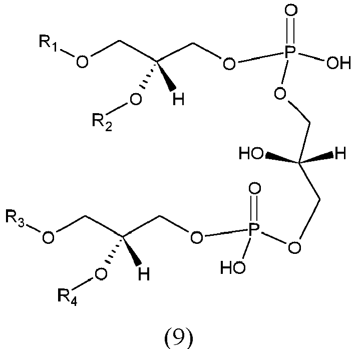 wherein
R₁, R₂, R₃ and R₄ are fatty acyls (CL, cardiolipins) with at least one of R₁, R₂, R₃ and R₄ being a MCFA, wherein in said MCFA n is preferably 1 or 2.

For example, CL with MCFA (C8:0, C10:0).

In one embodiment, the lipid compound is selected from compounds of formula 10 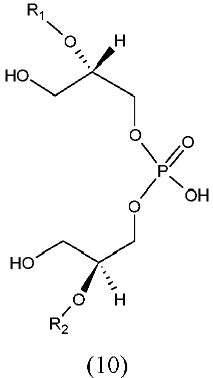 wherein
R₁ and R₂ are fatty acyls (BMP, bis(monoacylglycero)phosphates) with at least one of R₁ and R₂ being a MCFA, wherein in said MCFA n is preferably 1 or 2.

For example, BMP with MCFA (C8:0, C10:0).

The use of the present invention preferably comprises determining the relative or absolute concentration of at least one lipid compound, as defined herein, in a sample, preferably at least one compound selected from compounds of formula 2 to 6.

In a preferred embodiment, at least two of the lipid compounds are used, wherein preferably at least one of said two lipid compounds is as defined herein, such as a compound of formula 2 to 6, or both lipid compounds are as defined herein.

In a preferred embodiment, the levels between at least two lipid compounds in a sample are compared, i.e the molar ratio of at least two lipid compounds is determined.
Preferably, thereby at least one of said at least two lipid compounds or both are as defined herein.

The use of the present invention also comprises the comparison of the levels of said lipids, or their relative or absolute concentrations, whereby at least one lipid compound is as defined herein, such as a compound of formula 1 to 10.

For example, the molar ratio of corresponding MCFA- and LCFA-containing lipid compounds is determined, preferably
C8:0-fatty acyl-CoA to C16:0-fatty acyl-CoA,
C8:0-fatty acyl-CoA to C18:0-fatty acyl-CoA
C10:0-fatty acyl-CoA to C16:0-fatty acyl-CoA,
C10:0-fatty acyl-CoA to C18:0-fatty acyl-CoA,
C8:0-fatty acid to C16:0-fatty acid,
C10:0-fatty acid to C16:0-fatty acid,
C8:0-acylcarnitine to C16:0-acylcarnitine,
C10:0-acylcarnitine to C16:0-acylcarnitine,
PC(8:0-10:0) to PC(16:0-18:1),
PC(10:0-10:0) to PC(16:0-18:1),
PC(16:0-8:0) to PC(16:0-18:1), and/or
PC(20:4-10:0) to PC(16:0-18:1).

The sample is preferably a biological sample from a human,
such as blood, plasma, serum, blood cells (preferably platelets), dried-blood spots, tissue samples from biopsies, cerebrospinal fluid, urine, saliva, or other cells, e.g. neuronal cells, cardiomyocytes.

A preferred sample are human platelets.

In platelets, preferably the lipid compound used is C8:0-CoA, C10:0-CoA, PC(10:0-8:0), PC(10:0/10:0), PC(16:0-8:0), and/or PC(10:0/20:4).

The disease is due to or accompanied by fatty acid oxidation disorder, which is due to mitochondrial or peroxisomal dysfunction.
The disease which is due to or accompanied by fatty acid oxidation disorder is preferably selected from
cardiovascular disease (CVD),
coronary artery disease (CAD),
stroke,
acute coronary syndrome (ACS),
chronic coronary syndrome (CCS),
heart failure.

Determining the relative or absolute concentration of the lipid compound(s) is preferably carried out using mass spectrometry.

More preferably, the mass spectrometry is electrospray ionization-tandem mass spectrometry (QqQ, QTRAP) or electrospray ionization-high-resolution accurate mass spectrometry (QTOF, Orbitrap, FTICR-MS).

In one embodiment, the mass spectrometry is preferably hyphenated to liquid chromatography and/or ion mobility spectrometry, or both.

In one embodiment, furthermore the relative or absolute concentration of at least one oxidized phospholipid is determined (in combination with at least one lipid compound as defined herein).
Examples for oxidized phospholipids (OxPLs) are
POVPC (1-palmitoyl-2-(5 '-oxo-valeroyl)-sn-glycero-3-phosphocholine),
PONPC (1-palmitoyl-2-(9-oxo-nonanoyl)-sn-glycero-3-phosphocholine),
PGPC (1-palmitoyl-2-glutaryl-sn-glycero-3-phosphocholine),
PAzPC (1-palmitoyl-2-azelaoyl-sn-glycero-3-phosphocholine),
PC 20:4-HDOHE,
PE 16:0p/HDOHE,
PE 16:0-HDOHE,
PC 18:0-HETE.

"HDOHE" refers to hydroxydocosahexaenoic acid and represents oxidized DHA i.e. FA22:6 (all isomers); "HETE" refers to hydroxyeicosatetraenoic acid and represents oxidized arachidonic acid i.e. FA20:4 (all isomers).

In one preferred embodiment, a panel of lipid compounds is used as the biomarker.
Thereby, at least one lipid compound of said panel is a lipid compound as defined herein, i.e. a lipid compound having a MCFA as fatty acyl residue.

Preferably, at least one of the following five lipid compounds is determined (is comprised in the panel):
PC(8:0/8:0),
PC(10:0-8:0),
PC(10:0/10:0),
PC(10:0-20:4), and
PC(8:0-16:0),
wherein, preferably, the sample are platelets,
and wherein preferably an upregulation of said lipid compounds is indicative for higher risk of disease or higher disease severity.

For example, an upregulation of said lipid compounds is indicative for higher risk of ACS or higher disease severity of ACS.

In a preferred embodiment, the lipid panel comprises more than one of said five lipid compounds, such as
PC(8:0/8:0),
PC(10:0-8:0),
PC(10:0/10:0),
PC(10:0-20:4), and
PC(8:0-16:0).

In said embodiment, furthermore (i.e. the panel comprises in addition to the 5 above lipid compounds)
PE(16:0-18:0), PC(16:0e/16:1) and/or PC(18:1e/16:0) are determined,
wherein, preferably, the sample are platelets,
and wherein preferably an upregulation of said lipid compounds is indicative for higher risk of disease or higher disease severity.
For example, an upregulation of said lipid compounds is indicative for higher risk of ACS or higher disease severity of ACS.

In said embodiment, furthermore at least one lipid selected from a phosphatidylcholine (PC), phosphatidylethanolamine (PE), diacylglycerol (DG) or triacylglycerol (TG), each with a docosahexaenoic acid fatty acyl side chain (FA22:6), is determined (i.e. is comprised in the panel),
and/or furthermore at least one of
PC(15:0-20:4),
PC(16:0-22:6),
PC(17:0-20:4),
PC(17:0-22:6),
PC(18:0-20:4),
PC(18:0-22:6),
PC(20:0-22:6),
PC(20:4-22:6),
PC(22:5-22:5),
PC(16:1e/20:4),
PC(20:0e/22:6),
LPC 18:0,
LPE 16:0,
LPE 18:0,
PE(18:1e/22:6),
PE(20:4-22:6),
DG(18:0-22:4),
TG(16:0-18:0-22:6),
TG(16:0-20:0-22:6),
TG(18:0-20:4-22:6), and/or
SM(d18: 1/22:0),
is/are determined (i.e. is comprised in the panel),
wherein, preferably, the sample are platelets,
and wherein preferably a downregulation is indicative for higher risk of disease, such as ACS, or higher disease severity, such as ACS.

In one embodiment, the panel of lipid compounds which are used as biomarkers comprises all of
PC(8:0/8:0),
PC(10:0-8:0),
PC(10:0/10:0),
PC(10:0-20:4),
PC(8:0-16:0),
PE(16:0-18:0),
PC(16:0e/16:1),
PC(18:le/16:0)
PC(15:0-20:4),
PC(16:0-22:6),
PC(17:0-20:4),
PC(17:0-22:6),
PC(18:0-20:4),
PC(18:0-22:6),
PC(20:0-22:6),
PC(20:4-22:6),
PC(22:5-22:5),
PC(16:1e/20:4),
PC(20:0e/22:6),
LPC 18:0
LPE 16:0,
LPE 18:0,
PE(18:1e/22:6),
PE(20:4-22:6),
DG(18:0-22:4),
TG(16:0-18:0-22:6),
TG(16:0-20:0-22:6),
TG(18:0-20:4-22:6), and
SM(d18:1/22:0),
wherein preferably an upregulation of PC(8:0/8:0), PC(10:0-8:0), PC(10:0/10:0), PC(10:0-20:4), PC(8:0-16:0), PE(16:0-18:0), PC(16:0e/16:1) and PC(18:1e/16:0) is indicative for higher risk of disease, such as ACS, or higher disease severity;
wherein more preferably an upregulation of PC(8:0/8:0), PC(10:0-8:0), PC(10:0/10:0), PC(10:0-20:4), PC(8:0-16:0), PE(16:0-18:0), PC(16:0e/16:1) and PC(18:1e/16:0) and a downregulation of the further lipid compounds is indicative for higher risk of disease, such as ACS, or higher disease severity.

In said embodiment, the sample are human platelets.

### Methods using the lipid biomarkers

As outlined above, the present invention provides a method for assessing the risk to develop an age-related disease due to fatty acid oxidation disorder.

Said method of the present invention comprises
(a) providing a sample obtained from a human,
(b) determining the relative or absolute concentration of at least one lipid compound in said sample,
wherein said lipid compound is as defined herein.

In one embodiment, the method further comprises
(c) comparing the levels between/determining the molar ratio of at least two lipid compounds in a sample.
In said step (c), the levels between at least two lipid compounds in a sample are compared, i.e the molar ratio of at least two lipid compounds is determined.
Preferably, thereby at least one of said at least two lipid compounds or both are as defined herein.

Preferably and as discussed above, the sample obtained from a human is blood, plasma, serum, blood cells (preferably platelets), dried-blood spots, tissue samples from biopsies, cerebrospinal fluid, urine, saliva, or other cells, e.g. neuronal cells, cardiomyocytes.

The risk assessment preferably comprises diagnosis, prognosis, prediction and/or monitoring of a disease which is due to or accompanied by fatty acid oxidation disorder.

Said disease due to or accompanied by fatty acid oxidation disorder is, as discussed above, due to mitochondrial or peroxisomal dysfunction.

Preferably and as discussed above, the disease due to or accompanied by fatty acid oxidation disorder is selected from
cardiovascular disease (CVD),
coronary artery disease (CAD),
stroke,
acute coronary syndrome (ACS),
chronic coronary syndrome (CCS),
heart failure.

Preferably and as discussed above, determining the relative or absolute concentration of the lipid compound(s) is carried out using mass spectrometry,
wherein, preferably, the mass spectrometry is electrospray ionization-tandem mass spectrometry (QqQ, QTRAP) or electrospray ionization-high-resolution accurate mass spectrometry (QTOF, Orbitrap, FTICR-MS),
and/or wherein, preferably, the mass spectrometry is hyphenated to liquid chromatography and/or ion mobility spectrometry, or both.

In one embodiment, the molar ratio of corresponding MCFA- and LCFA-containing lipid compounds is determined, preferably
C8:0-fatty acyl-CoA to C16:0-fatty acyl-CoA,
C8:0-fatty acyl-CoA to C18:0-fatty acyl-CoA
C10:0-fatty acyl-CoA to C16:0-fatty acyl-CoA,
C10:0-fatty acyl-CoA to C18:0-fatty acyl-CoA,
C8:0-fatty acid to C16:0-fatty acid,
C10:0-fatty acid to C16:0-fatty acid,
C8:0-acylcarnitine to C16:0-acylcarnitine,
C10:0-acylcarnitine to C16:0-acylcarnitine,
PC(8:0-10:0) to PC(16:0-18:1),
PC(10:0-10:0) to PC(16:0-18:1),
PC(16:0-8:0) to PC(16:0-18:1), and/or
PC(20:4-10:0) to PC(16:0-18:1).

In one embodiment, furthermore the relative or absolute concentration of at least one oxidized phospholipid is determined (in combination with at least one lipid compound as defined herein). Examples for oxidized phospholipids (OxPLs) are
POVPC (1-palmitoyl-2-(5'-oxo-valeroyl)-sn-glycero-3-phosphocholine),
PONPC (1-Palmitoyl-2-(9-oxo-nonanoyl)-sn-glycero-3-phosphocholine),
PGPC (1-palmitoyl-2-glutaryl-sn-glycero-3-phosphocholine),
PAzPC (1-palmitoyl-2-azelaoyl-sn-glycero-3-phosphocholine),
PC 20:4-HDOHE,
PE 16:0p/HDOHE,
PE 16:0-HDOHE,
PC 18:0-HETE.

As discussed above, "HDOHE" refers to hydroxydocosahexaenoic acid and represents oxidized DHA i.e. FA22:6 (all isomers); "HETE" refers to hydroxyeicosatetraenoic acid and represents oxidized arachidonic acid i.e. FA20:4 (all isomers).

In one preferred embodiment, a panel of lipid compounds is used.
Thereby, at least one lipid compound of said panel is a lipid compound as defined herein, i.e. a lipid compound having a MCFA as fatty acyl residue.

In said embodiment and as discussed above, at least one of the following 5 lipid compounds is determined (is comprised in the panel):
PC(8:0/8:0),
PC(10:0-8:0),
PC(10:0/10:0),
PC(10:0-20:4), and
PC(8:0-16:0),
wherein, preferably, the sample are platelets,
and wherein preferably an upregulation of said lipid compounds is indicative for higher risk of disease or higher disease severity.
For example, an upregulation of said lipid compounds is indicative for higher risk of ACS or higher disease severity of ACS.

In a preferred embodiment, the lipid panel comprises more than one of said five lipid compounds, such as
PC(8:0/8:0),
PC(10:0-8:0),
PC(10:0/10:0),
PC(10:0-20:4), and
PC(8:0-16:0).

Preferably, furthermore (i.e. the panel comprises in addition to the 5 above lipid compounds)
PE(16:0-18:0), PC(16:0e/16:1) and/or PC(18:1e/16:0) are determined,
wherein, preferably, the sample are platelets,
and wherein preferably an upregulation of said lipid compounds is indicative for higher risk of disease or higher disease severity.

For example, an upregulation of said lipid compounds is indicative for higher risk of ACS or higher disease severity of ACS.

In said embodiment, furthermore at least one lipid selected from a phosphatidylcholine (PC), phosphatidylethanolamine (PE), diacylglycerol (DG) or triacylglycerol (TG), each with a docosahexaenoic acid fatty acyl side chain (FA22:6), is determined (i.e. is comprised in the panel),
and/or furthermore at least one of
PC(15:0-20:4),
PC(16:0-22:6),
PC(17:0-20:4),
PC(17:0-22:6),
PC(18:0-20:4),
PC(18:0-22:6),
PC(20:0-22:6),
PC(20:4-22:6),
PC(22:5-22:5),
PC(16:1e/20:4),
PC(20:0e/22:6),
LPC 18:0,
LPE 16:0,
LPE 18:0,
PE(18:1e/22:6),
PE(20:4-22:6),
DG(18:0-22:4),
TG(16:0-18:0-22:6),
TG(16:0-20:0-22:6),
TG(18:0-20:4-22:6), and/or
SM(d18: 1/22:0),
is/are determined (i.e. is comprised in the panel)
wherein, preferably, the sample are platelets,
and wherein preferably a downregulation is indicative for higher risk of disease, such as ACS, or higher disease severity, such as ACS.

In one embodiment, the method according to the present invention is carried out with other clinical markers.
Said clinical markers comprise
cardiac troponins (Troponin I and T),
(high-sensitivity, hs) C-reactive protein,
ApoB,
Lp(a),
LDL-C (LDL-Cholesterol), LDL-P (LDL-particle number),
CETP,
Lp-PLA2,
sICAM-1,
IL-6, IL-18,
SAA,
MPO,
sCD40,
oxidized LDL,
GPx1 activity,
nitrotyrosine,
homocysteine,
cystatin C,
(atrial and B-type) natriuretic peptides (ANP and BNP),
ADMA,
MMP-9,
TIMP-1,
fibrinogen,
D-dimer,
TPA/PAI-1,
blood pressure,
endothelial dysfunction,
arterial stiffness,
serum amyloid protein A,
TNFalpha,
APO-1,
urinary and plasma isoprostanes,
urinary biopyrrins,
plasma malondialdehyde,
heart type fatty acid binding protein,
myosin light-chain kinase I,
myocardial-bound creatine kinase,
ST2,
collagen propeptides,
galectin-3,
chromogranin,
adiponectin,
osteoprotegerin,
growth differentiation factor 15,
urinary thromboxanes, MCP-1,
plasma ceramides (Cer(d18:1/16:0), Cer(d18:1/18:0), Cer(d18:1/20:0), and Cer(d18:1/24:1), in addition to their ratios with Cer(d18:1/24:0)), sphingosine-1-phosphate/Cer(d18:1/24:1).

### Further description of preferred embodiments

Lipidomics has shown great potential to yield new biomarkers for clinical diagnostics, see for example, Ekroos et al. (2020), WO 2020/115288 A1; WO 2011/063470 A1 and US 2020/0088747 A1; WO 2014/188202 A1; US 2019/0086386 A1. Such methods are based on the monitoring of a combination of specific lipid concentrations, usually by mass spectrometry-based analytical techniques, in a sample from a human subject and comparing such concentrations to those of a control group, often corresponding samples of a healthy control. Specific lipids can be considered as useful biomarkers if their concentrations in a certain test group is increased or decreased as compared to the control, based on adequate statistical methods. Due to comprehensive measurement of a large number of lipids in untargeted lipidomics, panels of lipids can be employed as biomarker network which often have shown improved predictive/diagnostic/prognostic quality. Such lipid biomarkers have most frequently been determined in plasma or serum of human subjects. Cellular components of blood are of great interest as well and in the context of CVD platelets are of significant interest as they play a major role in the genesis of the disease.

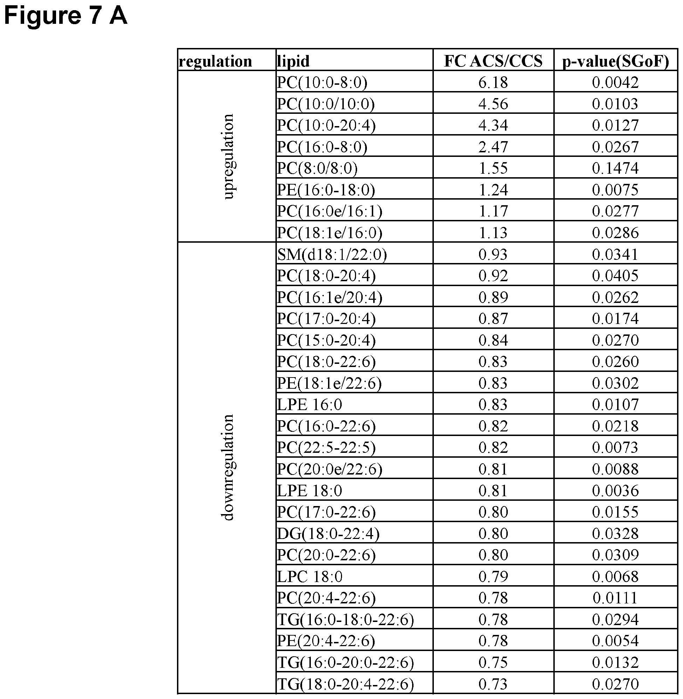

Platelets are critically involved in the pathophysiology of acute coronary syndrome (ACS). Enhanced platelet hyperreactivity is a risk factor for thromboischemic events in patients with coronary artery disease (CAD). Patients with enhanced platelet activation are at increased risk to develop adverse cardiovascular events including myocardial infarction, ischemic stroke, and death. We found that the platelet lipidome is significantly altered in patients with acute (ACS) when compared to chronic coronary syndrome (CCS) patients (see table of Figure 7A).

Platelets have a high energy consumption, and in spite of having a small number of mitochondria, they have a high rate of ATP turnover (Wang *et al.,* 2017). It has been reported that approximately 60% of ATP is derived from glycolysis during the resting state of platelets, and oxidative phosphorylation supplies the remaining amount of ∼30-40% energy. During platelet activation the energy demand is further elevated. Mitochondria act as the main energy suppliers in platelets during thrombus formation, which has been reversely testified by inhibiting mitochondrial respiration using pharmacological antagonists to the electron transport chain such as nitric oxide, antimycin A and cyanide (Wang *et al.*, 2017). If glycolytic pathways do not provide sufficient energy supply, fatty acids can serve as energy carriers and can provide acetyl-CoA for the TCA cycle by mitochondrial β-oxidation. For this purpose, triglyceride pools of platelets must be hydrolyzed to free fatty acids. To support this process, lipid-uptake in form of LDL or oxidized LDL from the plasma environment via LDL-receptors (LDL) or scavenger receptors (OxLDL) can feed into this process. For β-oxidation, the fatty acids must be transferred into mitochondria which occurs via acyl-carnitine shuttle. Free fatty acids are activated into acyl-CoAs by acyl-CoA synthetases (ACS) (Grevengoed *et al.*, 2014), converted into corresponding acyl-carnitines e.g. by carnitine-palmitoyl transferase 1 and back into the corresponding acyl-CoAs in the inner mitochondrial membrane by carnitine-palmitoyl transferase 2.

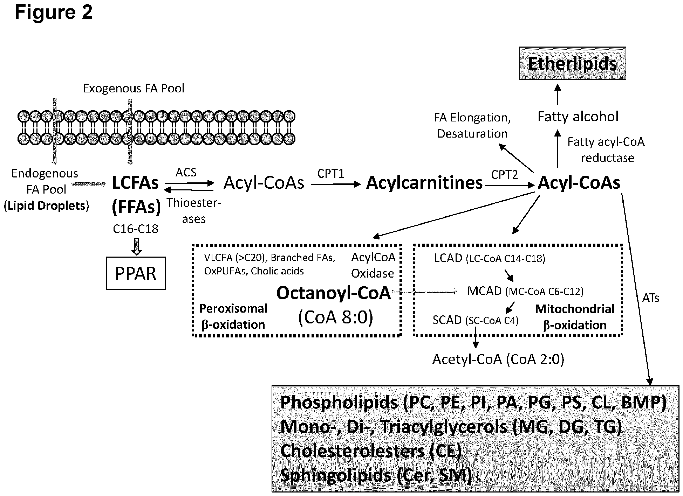

The regulation of mitochondrial β-oxidation depends on cellular energy status (Grevengoed *et al.,* 2014). When ATP levels are low, acyl-CoAs are transported into the mitochondria by CPT1. Mitochondrial β-oxidation of fatty acyl-CoAs is the major route of FA degradation, but very-long-chain FAs and branched-chain FAs are poorly oxidized in mitochondria and instead are degraded in peroxisomes. The β-oxidation capability of peroxisomes terminates at medium-chain acyl-CoAs and produces chain-shortened acyl-CoAs and acetyl- and propionyl-CoAs, which are transported out of the peroxisome as short to medium-chain acyl-carnitines to be completely oxidized in the mitochondria. Depending on its chain length, the acyl-CoA is converted to the corresponding carnitine ester by one of two peroxisomal enzymes, carnitine acetyltransferase or carnitine octanoyltransferase (Grevengoed *et al.*, 2014). Figure 2 shows the acyl-CoA metabolism.

Mitochondrial dysfunction exists in several age-related diseases (Haas 2019). Also platelet mitochondrial dysfunction has been reported in human diseases, such as diabetes mellitus, sepsis and neurodegenerative disorders (Wang *et al.,* 2017). It can be hypothesized that in such cases increased energy demand is then supplied by peroxisomal β-oxidation. Peroxisomal β-oxidation has usually a preference for very-long-chain acyl-CoAs (but can also accept long-chain acyl CoAs) which begin to be oxidized in peroxisomes, releasing acetyl-CoAs, until they are chain-shortened to 8 carbons (octanoyl-CoA, CoA 8:0), which complete their oxidation in the mitochondria. However, if mitochondria are dysfunctional, octanoyl-CoA (C8:0-acyl-CoA, CoA 8:0) might accumulate. In severe cases of accumulation of C8:0-acyl-CoA, also C10:0-acyl-CoA (CoA 10:0) and/or C12:0-acyl-CoA (CoA 12:0) accumulate.

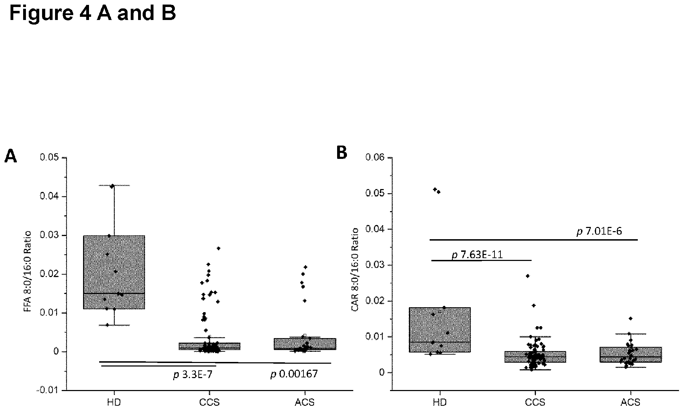

Mitochondrial dysfunction might be related to MCAD (medium-chain acyl-CoA dehydrogenase) which would lead to enhanced levels of C6-, C8-, and 10:0-acyl-CoAs (CoA 6:0, CoA 8:0, CoA 10:0). Genetic MCAD deficiency is tested in newborn screening for inborn errors of metabolism (Wang *et al.,* 2017; Li *et al.,* 2019). It is diagnosed in blood (spots) by enhanced levels of C6-, C8-, C10-, and C10:1-acylcarnitines (Pasquali *et al.*, 2018; Merritt and Chang, 2000). Multiple FAO enzyme deficiencies are observed in MADD (multiple acyl-CoA dehydrogenase deficiency) (Prasun, 2020). Formal clinical diagnostic criteria for multiple acyl-CoA dehydrogenase deficiency (MADD) have not been established, but multiple acylcarnitine species (C4, C5, C8, and other higher acylcarnitine) are above the common cutoff values. Our results of UHPLC-MS/MS measurements of the lipid profile in platelets of CAD patients indicate that several lipid compounds with medium chain fatty acids are increased due to fatty acid oxidation (FAO) disorders owing to mitochondrial or peroxisomal disorders. Enhanced levels of FA 16:0 result in decreased C8:0/C16:0 molar ratios for acyl carnitines and free fatty acids in healthy donors (HD) compared to CAD patients (CCS and ACS) (Figure 4). LCFAs, such as C16:0 FA, feed into the FAO, leading to chain shortened acyl CoAs.

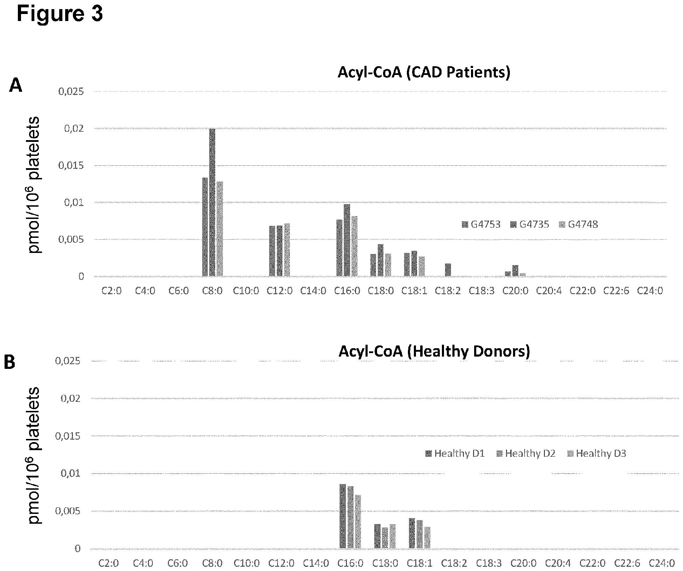

The targeted UHPLC-ESI-MS/MS analysis of platelet acyl-CoAs of healthy donors and CAD patients revealed significant differences in their acyl-CoA profiles (as shown in Figure 3). C8:0- and C12:0-acyl-CoAs (CoA 8:0, CoA 12:0) were solely detected in platelets of patients while the usually major long chain acyl-CoAs (C16-C18; CoA 16:0, CoA 18:0, CoA 18:1) are essentially at the same levels in all platelet samples, both from healthy donors and patients.

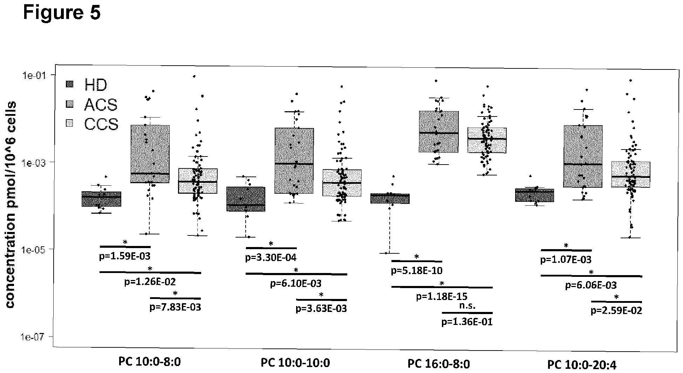

To avoid excessive accumulation and stop of mitochondrial/peroxisomal β-oxidation, C8:0-, C10:0- and C12:0-acyl-CoAs are fed into interconnected pathways (see Figure 2). They can be converted to C8:0-, C10:0- and C12:0-acyl-carnitines by peroxisomal carnitine octanoyltransferase (CROT) and transferred to platelet cytosol. They can be hydrolyzed by thioesterases yielding free C8:0-FA, C10:0-FA and C12:0-FA as well as free CoA-SH needed for further β-oxidation cycles. Last but not least, they can be reesterified into corresponding phospholipids (e.g. PC(8:0/8:0), PC(8:0-10:0), PC(10:0/10:0), PC(16:0-8:0); PC(10:0-20:4)), diacylglycerols (e.g. DG(8:0-10:0); DG(10:0/10:0)), triglycerides (TGs), cholesterylesters (CEs), sphingolipids (SPLs). These medium-chain fatty acyl-PCs are only found in platelets of subjects suffering to some extent from CAD and are therefore highly suitable biomarkers of disease for CAD (see Figure 5).

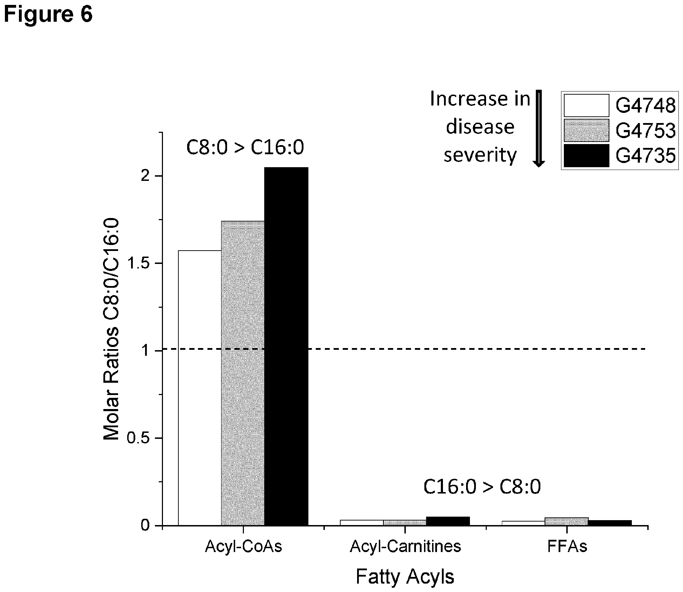

Since absolute levels of C8-acyls may depend to some extent on the general lipid metabolism of a subject, the molar ratios can be more suitable for risk stratification. For example, we can see that the C8:0/C16:0-CoA ratio increases in order of disease severity (G4748, mild, molar ratio = 1.57; G4753, medium, molar ratio = 1.74; G4735, severe, molar ratio = 2.05) (see Figure 6). It means C8:0 CoA is present in excess as compared to C16:0-CoA which may be the reason why we find these C8:0-fatty acyls biotransformed into PCs. In contrast, in case of acyl-carnitines and free fatty acids (FFA) C16:0 dominates over C8:0-fatty acyls and the correlation with disease severity is less pronounced.

### Lipid panel

The inventors have found that significant changes in the platelet lipidome occur in patients with ACS as compared to CCS and healthy donors.
We characterized the platelet lipidome in a large cohort of patients with CAD by untargeted lipidomics technology. We identified 928 lipids extracted from isolated platelets. Main lipid categories were defined as glycerophospholipids, glycerolipids, sphingolipids, fatty acyls, and sterol lipids. Comparison of patients that presented with ACS compared to those with stable CCS revealed that seven lipids were significantly upregulated in ACS. Upregulated lipids comprised primarily lipids belonging to the category of glycerophospholipids. Several of the upregulated lipids comprised at least one medium chain fatty acyl side chain. Furthermore, they comprised primarily glycerophospholipids (phosphocholines and phosphoethanolamine) having high degree of saturation, and/or ether linkage implying a role in the pathophysiology of ACS.

Among the identified upregulated glycerophospholipids, PC18:0 was found in *in vitro* studies to exhibit substantial prothrombotic effects. Our findings imply that upregulation of glycerophospholipids with MCFAs in platelets contribute to the pathophysiology of ACS by promoting platelet-dependent thrombus formation.

Besides enzymes for β-oxidation, peroxisomes harbor enzymes for α-oxidation and ether lipid synthesis. Ether phospholipid (plasmalogen) synthesis can be enhanced when peroxisomal activity is elevated and/or peroxisomal activity induced. They have been reported to be involved in the protection against oxidative stress and have important anti-oxidative function in biomembranes (Gorgas *et al.,* 2006). In fact, Figure 7A shows that some ether lipids, viz. PC(18:1e/16:0) (PC34:1e) and PC(16:0e/16:1) (PC32:1e), are significantly upregulated in ACS as compared to CCS.

Besides these upregulated lipids, a number of lipids were downregulated in ACS as compared to CCS. The majority of these downregulated lipids are glycerolipids (GLs) and glycerophospholipids (GPLs), in particular PCs and PEs, with PUFA side chains (arachidonic acid FA 20:4, eicosapentaenoic acid EPA FA 20:5, and docosahexaenoic acid DHA, FA 22:6). It is striking that many have DHA side chain. DHA has long been recognized for its beneficial effects in humans. While eicosanoids derived from GPLs with arachidonic acid (FA 20:4) side chain have strong proinflammatory properties, those produced from GPLs with eicosapentaenoic acid (EPA, FA 20:5) or docosahexaenoic acid (DHA, FA 22:6) fatty acyl residues are anti-inflammatory or pro-resolving inflammation. DHA is an essential component of cell membranes in some human tissues and regulates membrane fluidity. In mammals, DHA is biosynthesized from the essential fatty acid α-linolenic acid, which must be exogenously supplied, via desaturase and elongase steps. Its final biosynthesis step requires one cycle of β-oxidation of FA 24:6 in the peroxisomes. The downregulation of these PUFA-containing lipids can be due to impaired biosynthesis or their modification due to oxidative stress.

We furthermore identified a number of PCs with odd-chain fatty acids (OCFAs) (C15:0 and C17:0) which are significantly down-regulated in ACS compared to CCS (PC15:0-20:4; PC17:0-20:4; PC17:0-22:6), see Figure 7A. These lipids had also poly-unsaturated fatty acid (PUFA) side chain as second acyl residue. The origin of odd chain lipids in mammalian may be surprising but they are most likely of dietary nature as they are present in trace levels in dairy fat and some fish and plants. In a recent publication, it was hypothesized that these odd-chain saturated fatty acids (OCFAs) are essential and have beneficial health effects (Venn-Watson *et al.*, 2020): Higher circulating concentrations of OCFAs, pentadecanoic acid (C15:0) and heptadecanoic acid (C17:0) were shown to be associated with lower risks of cardiometabolic diseases, and higher dietary intake of OCFAs was associated with lower mortality. Interestingly, C15:0 was described in this work as a dual, partial PPAR α/δ agonist and able to repair mitochondrial function. So, these lipids which indicate a nutritional factor or changes in the microbiome contribute to the lipidomic distinction of patient groups in accordance to disease severity. Since the relevant PCs with odd-chain fatty acids also carry PUFAs, the effect is not easy to deconvolute from the one of downregulated lipids with PUFA (arachidonic acid, FA 20:4, and docosahexaenoic acid, FA 22:6) side chains.

The downregulation of LPC18:0 and LPE16:0 (in Figure 7A) could be a result of their re-esterification in phospholipids. For example, it has been reported by Slatter *et al.* (2016) that oxidized fatty acids (eicosanoids) are reesterified into oxidized phospholipids. Many oxidized PEs were detected in platelets and they could result from LPEs through acyltransferase (AT) activity. Similarly, LPCs could be reesterified by AT activity (Bakken *et al.,* 1992).

The upregulation of PCs with medium chain fatty acids (C8:0 and C10:0), the formation of oxidized PCs, the down regulation of PCs with PUFA side chain, the upregulation of ether lipids, the downregulation of LPCs in ACS as compared to CCS indicates that these lipids (as shown in Figure 7A) are highly suitable as biomarkers for the assessment of the risk of cardiovascular disease and disease severity. Oxidized fatty acids (from oxidative stress or thromboinflammation in platelets of patients) are reesterified into oxidized phospholipids which are built into the membrane and present a procoagulant surface for platelet aggregation (Slatter *et al.,* 2016). Medium chain fatty acyls (C8:0-C14:0), in particular C8:0 and C10:0-fatty acyls, accumulate in platelets of patients (due to fatty acid oxidation disorder) and are reesterified into PCs with medium chain fatty acyl (e.g. PC(10:0-10:0), PC(10:0-8:0), PC(16:0-8:0)). The PCs with C8:0 and C10:0 acyl chains augmented platelet aggregation and platelet-dependent thrombus formation under flow substantially. Both pathological mechanisms of membrane remodeling in CAD patients may generate platelet hyperreactivity which increases the risk of cardiovascular events, i.e. of myocardial infarction and stroke. By analyzing those lipid biomarkers in subjects with enhanced risk (age > 50 yrs, hyperlipidemia, familial CVD history, etc) early indicators for professional disease management (change of life style, therapeutic interference) are available.

The following examples and drawings illustrate the present invention without, however, limiting the same thereto.

### BRIEF DESCRIPTION OF THE DRAWINGS

**Figure 1** *Lipid categories and major lipid classes.*
   As defined by the lipid map consortium (https://www.lipidmaps.org/).
**Figure 2** *Acyl-CoA metabolism.*
   Exogenous (plasma lipids) and endogenous lipid pools (e.g. lipid droplets) represent reservoirs for free fatty acids, in particular long chain fatty acids (LCFAs) with C16-C18 carbon chain. They are activated into acyl-CoAs by acyl-CoA synthetases (ACS). Thioesterases, on the other hand, can hydrolyze acyl-CoAs to release free CoA-SH and free fatty acids. In the fatty acid oxidation (FAO) pathways, CPT1 (carnitine palmitoyltransferase-1) converts long-chain acyl-CoAs to acylcarnitines that can enter mitochondria for β-oxidation. The initial step of each FAO cycle is performed by dehydrogenases which are selective for carbon chain length, LCAD (long chain acyl CoA dehydrogenase) oxidises LC-CoAs with 14-18 carbons, MCAD (medium chain acyl CoA dehydrogenase) and SCAD (short chain acyl CoA dehydrogenase) oxidize medium (6-12 carbons) and short chain acyl-CoAs (4 carbons). Very-long-chain acyl-CoAs, on the other hand, are oxidized (by Acyl-CoA-Oxidase) in peroxisomes, releasing acetyl-CoAs, until they are chain-shortened to 8 carbons leading to octanoyl-CoA (MC-CoA), which is transferred to mitochondria where its FAO oxidation is completed. Fatty acyl-CoA reductase converts acyl-CoAs to fatty alcohols that will be incorporated into ether lipids. Acyl-CoA acyltransferases (ATs) incorporate fatty acids into complex lipids such as glycerophospholipids (PC, PE, etc), glycerolipids (MG, DG, TG), cholesterolesters, and sphingolipids. The carbon chain of long chain acyl CoAs can be extended by desaturases and elongases leading to very long chain acyl-CoAs.
**Figure 3** *Differences in acyl-CoA profiles between platelet acyl-CoAs of healthy donors and CAD patients.*
   Acyl-CoA profile in platelets of healthy donors (D1, D2, D3) and CAD patients with severe (G4735), intermediate (G4753), mild (G4748) clinical symptoms are shown. It can be seen that C8:0- and C12:0-acyl-CoAs are solely detected in platelets of CAD patients while the usually major long chain acyl-CoAs (C16-C18) are essentially at same levels in all platelet samples, both from healthy donors and patients.
**Figure 4** *C8-acylcarnitines and free C8-fatty acids in platelets of CAD patients.*
   Comparison of ratios of concentrations (pmol/10⁶ platelets) of (A) C8:0/C16:0 acyl-carnitines and (B) C8:0/C16:0 free fatty acids (FFA) in platelets of healthy donors (HD) (n=11), patients with chronic coronary syndrome (CCS) (n=81) and acute coronary syndrome (ACS) (n=26).
**Figure 5** *Medium chain fatty acyl-PCs as biomarkers of disease for CAD.*
   Comparison of concentrations (pmol/10⁶ platelets) of PC(10:0-20:4), PC(16:0-8:0), PC(10:0-10:0) and PC(10:0-8:0) in platelets of healthy donors (HD) (n=11), patients with chronic coronary syndrome (CCS) (n=81) and acute coronary syndrome (ACS) (n=26). A p-value of < 0.05 is considered as significant. MCFA-PCs in HD were <LOD (S/N=3), thus peak intensities were obtained by gap filling to enable statistical analysis. For gap filling, peak maxima for the precursor m/z-values were extracted within the expected retention time window of each MCFA-PC. Concentration values were then calucalted by relative quantification using a class-specific isotopically labelled internal standard.
**Figure 6** *Molar ratios of C8:0 and C16:0 fatty acyls as biomarkers for risk stratification and for determining disease severity.*
   Molar ratios of C8:0 and C16:0 fatty acyls in platelets as determined by LC-MS/MS. Acyl-CoAs are the activated fatty acids used for biosynthesis of phosphoglycero lipids (such as PCs) and glycerolipids, respectively, while acyl-carnitines are fatty acid metabolites used as shuttle/for transport (via organic cation transporter to/from mitochondria and free fatty acids (FFA) are precursors of the other fatty acyls. It can be seen that the molar ratios are reversed, i.e. higher levels of C8:0-acyl-CoA compared to C16:0-acyl-CoA and C16:0 dominance over C8:0 for the other two fatty acyl lipid classes (acyl-carnitines and FFA).

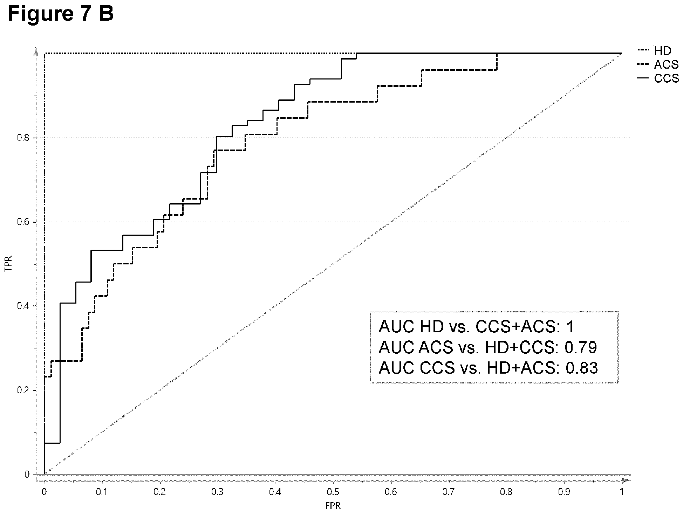

**Figure 7** *Lipidomic alteration shows a wide range in acute coronary syndrome.*
   A, Table showing semi-quantitatively regulation (log2 FC ACS/CCS) of significantly altered lipids in CAD patients. Seven lipids were significantly upregulated in ACS (SGoF p<0.05, FDR<5%) exclusively belonging to the category of glycerophospholipids. PC18:0 showed highest increase in ACS Patients (6-fold) compared to CCS. In contrast 21 lipids were downregulated in ACS compared to CCS (SGoF p<0.05, FDR<5%).
   B, Receiver operating characteristic (ROC) curves for biomarker panel specified in Table of Fig 7A. AUC, area under curves: TPR = true positive rate (sensitivity); FPR = false positive rate (1-specificity); HD = healthy donors; ACS = acute coronary syndrome; CCS = chronic coronary syndrome

### EXAMPLES

### Example 1 UHPLC-ESI-MS/MS Method for the determination of fatty acyl CoAs

### 1. Material and Methods

### 1.1 Materials

Fatty acyl-coenzyme A (acyl-CoA) standards were purchased from Avanti Polar Lipids (Alabaster, AL, USA) or Sigma-Aldrich (supplied by Merck, Taufkirchen, Germany). Fully ¹³C-labelled yeast extract of more than 2 × 10⁹ Pichia pastoris cells was obtained from ISOtopic Solutions (Vienna, Austria). Standards were dissolved in 50% methanol to give stock solutions of 5 mg/mL except for C16(d4):0-CoA (1 mg/mL). Internal standard solution was prepared by reconstituting dry Pichia pastoris cell extract with 2.0 mL methanol. Solutions were kept at -80 °C until further use. Trimethylsilyl diazomethane (TMS-DM) was obtained from Acros Chemicals (supplied by VWR, Bruchsal, Germany).

### 1.2 Sample preparation

Aliquots of 5 × 10⁸ platelets, isolated from human blood of CAD patients and healthy donors according to literature procedures (Chatterjee *et al.,* 2017), were employed. Acyl-CoAs were extracted by a combination of liquid and solid phase extraction (SPE). Thus, 80 µL of internal standard solution and 750 µL of acetonitrile/isopropanol (3:1, v/v) were added to the platelet pellets. Samples were homogenized for 1 min with a cell disruptor (Branson Sonifier B15, Branson Instruments Co., Stamford, USA). 250 µL of 0.1 M KH₂PO₄ buffer (pH 6.7) were added and samples were homogenized for 1 min. After centrifugation (10 min, 1835 g, 4 °C), the supernatant was acidified by addition of 250 µL of acetic acid and was loaded onto Oasis WAX SPE cartridge (Waters, Milford, MA, USA), which was pre-conditioned with 1 mL of methanol/water/acetic acid (3:1:1, v/v/v). After washing twice with 1 mL of this solution and twice with 1 mL of 50% (v/v) methanol Acyl-CoAs were eluted with 1.5 mL of isopropanol/methanol/10% ammonia (6:3:1, v/v/v). The eluates were dried under nitrogen at R.T. in a Genevac EZ2 system (SP Scientific, Ipswich, UK).

The dried extracts were reconstituted with 100 µL of methanol. 1 mL of the upper layer of *tert-*butyl methyl ether/methanol/water (10:3:2.5, v/v/v) and 100 µL of TMS-DM (2M in hexane) were added. The solutions were gently mixed and allowd to react for 30 min at room temperature. Then, 5 µL of acetic acid were added to quench the reaction. Samples were dried under N₂ in an evaporator (Genevac EZ-2) and kept at -80°C until analysis. Dried samples were first reconstituted with 50 µL of DMSO, diluted with 50 µL of H₂O and used for LC-MS/MS analysis.

### 1.3 UHPLC-ESI-MS/MS analysis

Separation was performed on a 1290 Infinity UHPLC system (Agilent Technologies, Waldbronn, Germany) with an ACQUITY UPLC CSH C18 Column (100 mm × 2.1 mm, 1.7 µm, 130 Å) and an ACQUITY UPLC CSH C18 VanGuard Pre-column (5 mm × 2.1 mm, 1.7 µm, 130 Å) (Waters, Milford, MA, USA). Mobile phase A was 10 mM ammonium acetate in water. Mobile phase B was methanol. Gradient elution (0.0 min, 10% B; 3 min, 10% B; 16 min, 100% B; 21 min, 100% B; 22 min, 10% B; 25 min, 10% B) was carried out at a flow rate of 0.4 mL/min and a constant column temperature of 50°C. Injection volume was 20 µL. Acyl-CoA detection was performed on a QTRAP 4500 mass spectrometer with a Turbo V source (Sciex, Concord, ON, Canada) operated with ESI probe. Data were acquired under the following parameters: collision gas (N₂) 6 psi, curtain gas (N₂) 30 psi, nebulizer gas (zero grade air) 50 psi; heater gas (zero grade air) 50 psi, ion source voltage 3500 V (positive mode) and source temperature 500 °C. SRM transitions were measured 30 s around the target retention time as specified in Table 1. The total cycle time was 0.6 s and dwell time was adjusted automatically. Analyst 1.7, Peak View and MultiQuant were used for data acquisition and data processing, respectively.

**Table 1. Acyl-CoAs analyzed by this method and compound-specific MS parameters ^{a, b}**

| Acyl-CoA | Q1 | Q3 | RT (min) | DP (V) | CE (V) | CXP (V) |
|---|---|---|---|---|---|---|
| CoA-SH | 838.2 | 275.1 | 7.84 | 80 | 45 | 20 |
| Malonyl | 924.2 | 361.1 | 8.01 | 80 | 45 | 20 |
| C2:0 | 866.2 | 303.1 | 8.00 | 80 | 45 | 20 |
| C3:0 | 880.2 | 317.2 | 8.77 | 80 | 45 | 20 |
| C4:0 | 894.2 | 331.2 | 9.53 | 80 | 45 | 20 |
| C6:0 | 922.3 | 359.2 | 10.76 | 80 | 45 | 20 |
| C8:0 | 950.3 | 387.2 | 11.50 | 80 | 45 | 20 |
| C10:0 | 978.3 | 415.3 | 13.14 | 80 | 50 | 25 |
| C12:0 | 1006.4 | 443.3 | 14.83 | 80 | 50 | 25 |
| C14:0 | 1034.4 | 471.3 | 15.34 | 80 | 50 | 25 |
| C16:0 | 1062.4 | 499.4 | 15.89 | 80 | 50 | 25 |
| C16(d4):0 | 1066.5 | 503.5 | 15.90 | 80 | 50 | 25 |
| C17:0 | 1076.4 | 513.4 | 16.12 | 80 | 50 | 25 |
| C18:0 | 1090.4 | 527.4 | 16.36 | 80 | 50 | 25 |
| C18:1 | 1088.4 | 525.4 | 16.02 | 80 | 50 | 25 |
| C18:2 | 1086.4 | 523.4 | 15.70 | 80 | 50 | 25 |
| C18:3 | 1084.4 | 521.3 | 15.39 | 80 | 50 | 25 |
| C20:0 | 1118.5 | 555.4 | 16.72 | 80 | 55 | 30 |
| C20:4 | 1110.4 | 547.4 | 15.99 | 80 | 55 | 30 |
| C20:5 | 1108.4 | 545.3 | 15.69 | 80 | 55 | 30 |
| C22:0 | 1146.5 | 583.5 | 17.02 | 80 | 55 | 30 |
| C22:6 | 1134.4 | 571.4 | 15.92 | 80 | 55 | 30 |
| C24:0 | 1174.5 | 611.5 | 17.27 | 80 | 55 | 30 |
| C25:0 | 1188.6 | 625.5 | 17.40 | 80 | 55 | 35 |
| U¹³C-C2:0 | 889.3 | 316.1 | 8.00 | 80 | 45 | 20 |
| U¹³C-C16:0 | 1099.5 | 526.5 | 15.89 | 80 | 50 | 25 |
| U¹³C-C18:0 | 1129.5 | 556.5 | 16.36 | 80 | 50 | 25 |
| U¹³C-C18:1 | 1127.5 | 554.5 | 16.02 | 80 | 50 | 25 |
| U¹³C-C22:0 | 1189.6 | 616.6 | 17.02 | 80 | 55 | 30 |
| U¹³C-C24:0 | 1219.7 | 646.6 | 17.27 | 80 | 55 | 30 |

| | | | | | | |
|---|---|---|---|---|---|---|
| ^{a} RT: retention time, DP: declustering potential, CE: collision energy, CXP: collision cell exit potential; ^{b} Entrance potential (EP) for all acyl-CoAs were 10 V. | | | | | | |

### 2. Results

The UHPLC-ESI-MS/MS method developed for acyl-CoA profiling allows the simultaneous determination of short-chain, medium-chain, long-chain and very long-chain acyl CoAs in one run. Its application for acyl-CoA profiling in human platelets of healthy donors revealed long-chain acyl CoAs (CoA 16:0, CoA 18:0 and CoA 18:1) as the dominant compounds (Figure 3B). The acyl Co profile of human platelets from coronary artery disease patients is different. While the levels of the long-chain acyl CoAs (CoA 16:0, CoA 18:0 and CoA 18:1) were very similar to the ones of healthy donors, very long-chain acyl CoAs (CoA 20:0) and medium-chain acyl CoAs (CoA 8:0, CoA 12:0) were also found (Figure 3A). They were not detected in platelets of healthy donors. Intriguingly, the molar ratio between CoA 8:0 and CoA 16:0 is above 1 in CAD patients which means that the MC-acyl CoA is dominating over the long chain CoA while for the free fatty acids and acyl carnitines the opposite is the case i.e. the long-chain fatty acid and acyl carnitine with C16:0 is the dominating lipid species in CAD patients leading to molar ratios between MC and LC free fatty acid and CAR fatty acyls below 1 (Figure 6). Acyl CoAs represent the metabolically active fatty acyls which represent activated fatty acids that are utilized in the pathways of glycerophospholipid and glycerolipid biosynthesis. Since MC-acyl CoAs are of significant concentration in platelets of CAD patients, they are incorporated in glycerophospholipids which explains why MC-PCs can be found in platelets of CAD patients.

### Example 2 Platelet lipidomics of clinical patient cohort

### 1. Material and Methods

### 1.1 Study population

One-hundred and thirty-nine patients with symptomatic CAD were enrolled in this study (Table 2). Patients were consecutively recruited into the study between February and May 2017 at the University Hospital of Tübingen. All patients were treated for symptomatic CAD and the severity of the disease was analyzed by coronary angiography. The study was approved by the local ethics committee (141/2018B02, 240/2018B02) and all patients gave written informed consent. The experiments were performed in accordance with the ethical standards as laid down in the Declaration of Helsinki. Venous blood samples were obtained within the first 24 hours after hospital admission.

**Table 2**

| | **All (n=139)** | **CCS Troponin - (n=100, 71.9%)** | **ACS Troponin + (n=39, 28.1%)** | **p-value** |
|---|---|---|---|---|
| Male, n (%) | 101 (72.7%) | 71 (71.0%) | 30 (76.9%) | 0.477 |
| Age, years (mean ± SD) | 70.6 (± 11.2) | 70.4 (± 10.4) | 71.2 (± 13.1) | 0.310 |
| Body mass index (mean ± SD) | 27.1 (± 4.7) | 27.3 (± 4.7) | 26.3 ± 4.8) | 0.170 |

| **Cardiovascular risk factors** | | | | |
|---|---|---|---|---|
| Arterial hypertension, n (%) | 109 (78.4%) | 80 (80.0%) | 29 (74.4%) | 0.473 |
| Hyperlipidemia, n (%) | 64 (46.0%) | 52 (52.0%) | 12 (30.8%) | 0.023 |
| Diabetes mellitus, n (%) | 41 (29.5%) | 27 (27.0%) | 14 (35.9%) | 0.307 |
| Current smoking, n (%) | 23 (16.5%) | 17 (17.0%) | 6 (15.4%) | 0.817 |
| Ex Smoking >6mo, n (%) | 23 (16.5%) | 17 (17.0%) | 6 (15.4%) | 0.817 |
| Obesity, n (%) | 65 (46.8%) | 54 (54.0%) | 11 (28.2%) | 0.006 |
| Atrial Fibrillation, n (%) | 33 (23.7%) | 24 (24.0%) | 9(23.1%) | 0.908 |
| Previous CABG, n (%) | 13 (9.4%) | 11 (11.0%) | 2 (5.1%) | 0.260 |
| Previous MI, n (%) | 29 (20.9%) | 25 (25.0%) | 4 (10.3%) | 0.043 |
| Renal function (GFR) (mean ± SD) | 74.9 (± 30.63) | 73.0 (± 25.7) | 79.6 (±40.6) | 0.577 |

| **Medication on admission** | | | | |
|---|---|---|---|---|
| Acetylsalicylic acid, n (%) | 112 (80.6%) | 83 (83.0%) | 29 (74.4%) | 0.257 |
| Clopidogrel, n (%) | 36 (25.9%) | 34 (34.0%) | 2(5.1%) | 0.001 |
| Ticagrelor, n (%) | 44 (31.7%) | 31 (31.0%) | 12 (30.8%) | 0.505 |
| Prasugrel, n (%) | 9 (6.5%) | 6 (6.0%) | 3 (7.7%) | 0.720 |
| Cangrelor, n (%) | 1 (0.7%) | 0 (0.0%) | 1 (2.6%) | 0.110 |
| Oral anticoagulants, n (%) | 33 (23.7%) | 28 (28.0%) | 5 (12.8%) | 0.048 |
| Angiotensin-converting enzyme inhibitors, n (%) | 71 (51.1%) | 53 (53.0%) | 18 (46.2%) | 0.468 |
| Angiotensin II receptor antagonists, n (%) | 32 (23.0%) | 25 (25.0%) | 7 (17.9%) | 0.366 |
| Aldosterone Antagonists, n (%) | 28 (20.1%) | 24 (24.0%) | 4 (10.3%) | 0.056 |
| Ca Channel Antagonists, n (%) | 38 (27.3%) | 31 (31.0%) | 7 (17.9%) | 0.111 |
| β-blockers, n (%) | 89 (64.0%) | 67 (67.0%) | 22 (56.4%) | 0.246 |
| Diuretics | 54 (38.8%) | 42 (42.0%) | 12 (30.8%) | 0.218 |
| Statins, n (%) | 92 (66.2%) | 76 (76.0%) | 16 (41.0%) | 0.001 |

| **Lipid profile parameters** | | | | |
|---|---|---|---|---|
| LDL-cholesterol (mg/dL) (mean ± SD) | 96.0 (± 32.9) | 94.1 (± 33.7) | 101.2 (± 30.5) | 0.193 |
| HDL-cholesterol (mg/dL) (mean ± SD) | 47.6 (± 16.1) | 48.8 (± 16.7) | 44.4 (± 14.0) | 0.225 |
| Triglycerides (mg/dL) (mean ± SD) | 150.4 (± 90.2) | 153.4 (± 89.6) | 142.3 (± 92.5) | 0.351 |
| Total cholesterol (mg/mL) (mean ± SD) | 161.2 (± 43.6) | 161.0 (± 42.7) | 161.7 (± 46.7) | 0.936 |
| **Platelets (10⁹/l) (mean ± SD)** | 225.1 (± 84.8) | 229.9 (± 72.7) | 230.8 (± 110.5) | 0.717 |

| **Disease** | | | | |
|---|---|---|---|---|
| Stable Angina | 75 (54.0%) | 75 (75.0%) | - | |
| Unstable Agina | 25 (18.0%) | 25 (25.0%) | - | |
| NSTEMI | 29 (20.9%) | - | 29 (74.4%) | |
| STEMI | 10 (7.2%) | - | 10 (25.6%) | |

| **Follow-up Events (12 months)** | | | | |
|---|---|---|---|---|
| Death | 7 (5.0%) | 6 (6.0%) | 1 (2.6%) | 0.375 |
| Myocardial Infarction | 0 (0.0%) | 0 (0.0%) | 0 (0.0%) | |
| Stroke | 0 (0.0%) | 0 (0.0%) | 0 (0.0%) | |

### 1.2 UHPLC-ESI- QTOF-MS/MS method

Platelets were isolated for lipidomic analysis as described (Schlotterbeck *et al.,* 2019; Cebo *et al.*, 2020). According to standardized protocols, lipid extraction from isolated platelets was performed with a monophasic extraction protocol (2-propanol/water) (Calderón *et al.,* 2019). A set of 16 isotopically labelled internal standards (ILIS, final concentrations of 4% SPLASH^{™} Lipidomix^{®} (14 ILIS), 100 ng/mL AA-d8, and 300 ng/mL C18 Cer (d18:1-18:0)) was added prior extraction. The lipid extracts were analyzed in three separate batches by a non-targeted lipidomic assay using liquid chromatography coupled to mass spectrometry (UHPLC-ESI- QTOF-MS/MS) using data independent acquisition with stepwise window acquisition of MS/MS spectra (SWATH) in positive and negative ion mode.

The open-source software MS-DIAL (Tsugawa *et al.,* 2015) was used for batch-wise data preprocessing including peak picking, alignment, and identification (structural annotation) by matching MS/MS spectra with in silica LipidBlast database. Based on peak tables of each batch from MS-DIAL a reference peak table was generated by an in-house developed batch alignment tool to combine lipidomic data from three batches to one data set. Using the reference peak table, batches were reanalyzed in MultiQuant (Sciex, Concord, Ontario, Canada) to extract peak heights of all reference list features. Preprocessed data were normalized using a combination of quality-control-based method systematic error removal using random forest (SERRF) (FiehnLab, Davis, California, USA) (Fan *et al.,* 2019) and ILIS-based method removal of unwanted variation random (RUVrandom) (Livera *et al.,* 2015) in RStudio 3.5.2 with the package NormalizeMets (De Livera *et al.,* 2018).

Normalized peak intensities were submitted to one-way analysis of variance (ANOVA) using JMP version 14.2.0 to identify peaks varying significantly (p< 0.05) between groups. To account for the problem of multiple hypothesis' testing, a false discovery rate (FDR) controlling procedure was further adopted to correct significance levels for FDR ≤ 5%. Therefore, we implemented sequential goodness of fit (SGoF) testing of lipid peak intensities using Myriads (Free Software Foundation, Inc., Boston, MA, USA). For statistical comparisons fold change was calculated as ratio between groups for each lipid and statistical evaluation and graphic output were performed with different software packages including JMP and RStudio (RStdio Inc., Boston, MA, USA). Lipidomic category tree was created using OmniGraffle 7.12 (The Omni Group, Seattle, WA, USA).

### 1.3 Statistical analysis

Patient data were analyzed using JMP^{®} version 14.2.0 (SAS Institute, Cary, North Carolina, USA). Non-normally distributed data were compared using the Mann-Whitney U test. Normally distributed data were analyzed using student's t-test. Mean values are presented as mean ± standard deviation. Correlations of normally distributed data were assessed by Pearson's correlation coefficient (r). Correlations of non-normally distributed data were analyzed using Spearman's rank correlation coefficient (*ρ*). Experimental data (mean ± SD) were analyzed using GraphPad Prism8 software (GraphPad Software, Inc., San Diego, CA, USA) at p < 0.05 statistical significance with two-way analysis of variance using Dunnett's post hoc test.

### 2. Results

### Characterization of the platelet lipidome in coronary artery disease

In the present study we analyzed the platelet lipidome by untargeted mass spectrometry in a large consecutive cohort of patients presenting with symptomatic CAD (n=139). The demographic details and characteristics are given in Table 2. Twenty-eight (28) % of the enrolled patients were diagnosed with troponin-positive ACS. After clearance of non-verifiable signals, we could identify 928 lipids extracted from isolated platelets. Among those lipids, 52% were classified as glycerophospholipids, 24% as glycerolipids, 19% as sphingolipids, 3.7% as fatty acyls, and 0.9% as sterol lipids. To evaluate whether the platelet lipidome is dependent on the acuity of the disease, we compared mean intensities of distinct lipids between patients with ACS and CCS. We found that among 928, seven lipids were significantly upregulated in ACS (p<0.05, FDR<5%) whereas 21 were downregulated compared to CCS (p<0.05, FDR<5%) (Figure 7A). Significantly upregulated lipids comprised exclusively lipids belonging to the category of glycerophospholipids (phosphocholines (PC, n=7; PC(18: 1e/16:0), PC(16:0e/16:1), PC(16:0-8:0), PC(10:0-20:4), PC(10:0/10:0), PC(10:0-8:0), PC(8:0/8:0)); phosphoethanolamine (PE, n=1; PE(16:0-18:0)). It is striking that several of these lipids carry at least one medium chain fatty acid (MCFA) side chain and share at least one of the following two characteristics: i) high degree of saturation, ii), and ii) alkenyl or alkyl ether linkage instead of acyl linkage in glycerophospholipids. Among those lipids that were downregulated, groups consist of
glycerophospholipids (n=16), comprising glycerophosphocholines (PC, n=11; PC(15:0-20:4), PC(16:0-22:6), PC(17:0-20:4), PC(17:0-22:6), PC(18:0-20:4), PC(18:0-22:6), PC(20:0-22:6), PC(20:4-22:6), PC(22:5-22:5), PC(16:le/20:4), PC(20:0e/22:6)); lyso-phospholipids (LPL, n=3; LPC 18:0, LPE 16:0, LPE 18:0); and glycerophosphoethanolamines (PE, n=2; PE(18: le/22:6), PE(20:4-22:6));
glycerolipids (n=4), comprising diglycerides (DG, n=1; DG(18:0-22:4)); triglycerides (TG, n=3; TG(16:0-18:0-22:6), TG(16:0-20:0-22:6), TG(18:0-20:4-22:6)), and
sphingolipids (n=1), comprising sphingomyelins (SM, n=1; SM(d18:1/22:0)) (Figure 7A).

Due to their significant regulation the MC-PCs can be alone, or in combination, used as biomarkers and biomarker panel, respectively, indicating the risk for developing coronary artery disease. Their performance and validity as biomarkers indicating disease severity is documented in Figure 7B. The AUCs of the ROC curves in said Figure 7B clearly document the excellent test qualities of this biomarker panel to indicate the risk for CAD (CCS and ACS) compared to HD (AUC=1.0) (note, MC-PCs are below the limit of detection in platelets of healthy donors). Furthermore, AUCs also suggest that the biomarker panel gives good tests for distinguishing between ACS and CCS (AUC of ACS vs. HD+CCS: 0.79; AUC of CCS vs. HD+ACS: 0.83). Hence, it is suggested to use this biomarker panel based on MC-PCs and supported by other significantly regulated lipids of Figure 7A for the stratification of the risk of CAD.

The features disclosed in the foregoing description, in the claims and/or in the accompanying drawings may, both separately and in any combination thereof, be material for realizing the invention in diverse forms thereof.

### REFERENCES

Bakken, A. M.; Farstad, M. The activities of acyl-CoA:1-acyl-lysophospholipid acyltransferase(s) in human platelets. Biochemical Journal 1992, 288, 763-770.
Calderón, C., et al., Comparison of simple monophasic versus classical biphasic extraction protocols for comprehensive UHPLC-MS/MS lipidomic analysis of Hela cells. Anal Chim Acta, 2019. 1048: p. 66-74.
Cebo, M., et al., Simultaneous targeted and untargeted UHPLC-ESI-MS/MS method with data-independent acquisition for quantification and profiling of (oxidized) fatty acids released upon platelet activation by thrombin. Anal Chim Acta, 2020. 1094: p. 57-69.
Chatterjee, M., et al., Regulation of oxidized platelet lipidome: implications for coronary artery disease. European Heart Journal, 2017. 38(25): p. 1993-2005.
De Livera, A.M., et al., NormalizeMets: assessing, selecting and implementing statistical methods for normalizing metabolomics data. Metabolomics, 2018. 14(5): p. 54.
Ekroos, K.; Lavrynenko, O.; Titz, B.; Pater, C.; Hoeng, J.; Ivanov, N. V. Lipid-based biomarkers for CVD, COPD, and aging - A translational perspective. Progress in Lipid Research 2020, 78, 101030.
Fahy E, Subramaniam S, Murphy RC, Nishijima M, Raetz CR, Shimizu T, Spener F, van Meer G, Wakelam MJ, Dennis EA. J Lipid Res. 2009 Apr;50 Suppl(Suppl):S9-14. doi: 10.1194/jlr.R800095-JLR200.
Fan, S., et al., Systematic Error Removal Using Random Forest for Normalizing Large-Scale UntargetedLipidomics Data. Analytical Chemistry, 2019. 91(5): p. 3590-3596.
Frossard, M., et al., Platelet function predicts myocardial damage in patients with acute myocardial infarction. Circulation, 2004. 110(11): p. 1392-7.
Gorgas, K.; Teigler, A.; Komljenovic, D.; Just, W. W. The ether lipid-deficient mouse: Tracking down plasmalogen functions. Biochimica et Biophysica Acta (BBA) - Molecular Cell Research 2006, 1763, 1511-1526.
Grevengoed, T. J.; Klett, E. L.; Coleman, R. A. Acyl-CoA Metabolism and Partitioning. Annual Review of Nutrition 2014, 34, 1-30.
Haas, R. H. Mitochondrial Dysfunction in Aging and Diseases of Aging Biology 2019, 48, 2-5.
Kautbally, S.; Lepropre, S.; Onselaer, M.-B.; Le Rigoleur, A.; Ginion, A.; De Meester de Ravenstein, C.; Ambroise, J.; Boudjeltia, K. Z.; Octave, M.; Wéra, O.; Hego, A.; Pincemail, J.; Cheramy-Bien, J.-P.; Huby, T.; Giera, M.; Gerber, B.; Pouleur, A.-C.; Guigas, B.; Vanoverschelde, J.-L.; Kefer, J.; Bertrand, L.; Oury, C.; Horman, S.; Beauloye, C. Platelet Acetyl-CoA Carboxylase Phosphorylation: A Risk Stratification Marker That Reveals Platelet-Lipid Interplay in Coronary Artery Disease Patients. JACC: Basic to Translational Science 2019, 4, 596-610.
Li, Y.; Zhu, R.; Liu, Y.; Song, J.; Xu, J.; Yang, Y. Medium-chain acyl-coenzyme A dehydrogenase deficiency: Six cases in the Chinese population. Pediatrics International 2019, 61, 551-557.
Livera, A.M.D., et al., Statistical Methods for Handling Unwanted Variation in Metabolomics Data. Analytical Chemistry, 2015. 87(7): p. 3606-3615.
Malehmir, M., et al., Platelet GPIbalpha is a mediator and potential interventional target for NASH and subsequent liver cancer. Nat Med, 2019. 25(4): p. 641-655.
Merritt, J.; Chang, I.: Medium-Chain Acyl-Coenzyme A Dehydrogenase Deficiency. In GeneReviews® [Internet]; Adam MP, A. H., Pagon RA, et al., editors, Ed.; University of Washington, Seattle: Seattle (WA), 2000 Apr 20 [Updated 2019 Jun 27].
Pasquali, M.; Longo, N.: Newborn screening and inborn errors of metabolism. In Tietz Textbook of Clinical Chemistry and Molecular Diagnostics; Rifai, N., Horvath, A. R., Wittwer, C. T., Eds.; Elsevier: St. Louis, Missouri, 2018.
Peng, B., et al., Identification of key lipids critical for platelet activation by comprehensive analysis of the platelet lipidome. Blood, 2018. 132(5): p. e1-e12.
Prasun, P.: Multiple Acyl-CoA Dehydrogenase Deficiency. In GeneReviews® [Internet]; Adam MP, A. H., Pagon RA, et al., editors, Ed.; University of Washington, Seattle: Seattle (WA), 2020 Jun 18.
Schlotterbeck, J., et al., Comprehensive MS/MS profiling by UHPLC-ESI-QTOF-MS/MS using SWATH data-independent acquisition for the study of platelet lipidomes in coronary artery disease. Anal Chim Acta, 2019. 1046: p. 1-15.
Slatter, David A.; Aldrovandi, M.; O'Connor, A.; Allen, Stuart M.; Brasher, Christopher J.; Murphy, Robert C.; Mecklemann, S.; Ravi, S.; Darley-Usmar, V.; O'Donnell, Valerie B. Mapping the Human Platelet Lipidome Reveals Cytosolic Phospholipase A2 as a Regulator of Mitochondrial Bioenergetics during Activation. Cell Metabolism 2016, 23, 930-944.
Tsugawa, H., et al., MS-DIAL: data-independent MS/MS deconvolution for comprehensive metabolome analysis. Nat Methods, 2015. 12(6): p. 523-6.
Venn-Watson, S.; Lumpkin, R.; Dennis, E. A. Efficacy of dietary odd-chain saturated fatty acid pentadecanoic acid parallels broad associated health benefits in humans: could it be essential? Scientific Reports 2020, 10, 8161.
Wang, L.; Wu, Q.; Fan, Z.; Xie, R.; Wang, Z.; Lu, Y. Platelet mitochondrial dysfunction and the correlation with human diseases. Biochemical Society Transactions 2017, 45, 1213-1223.
Witte, A., et al., The chemokine CXCL14 mediates platelet function and migration via direct interaction with CXCR4. Cardiovasc Res, 2020.
Zhao, L.; Huang, Y.; Lu, L.; Yang, W.; Huang, T.; Lin, Z.; Lin, C.; Kwan, H.; Wong, H. L. X.; Chen, Y.; Sun, S.; Xie, X.; Fang, X.; Yang, H.; Wang, J.; Zhu, L.; Bian, Z. Saturated long-chain fatty acid-producing bacteria contribute to enhanced colonic motility in rats. Microbiome 2018, 6, 107.

## Claims

1. Use of a lipid compound comprising at least one medium chain fatty acid (MCFA) of general formula 1 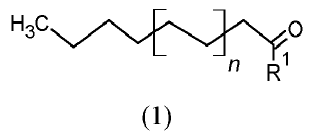 wherein
n is 0, 1, 2 or 3, preferably 1 or 2,
and
R₁ is -OH or a lipid backbone of a lipid selected from fatty acyls, glycerolipids, glycerophospholipids, sphingolipids and sterol lipids, preferably from fatty acyls, glycerolipids, and glycerophospholipids,
wherein said MCFA is optionally substituted and/or unsaturated,
as biomarker for assessing the risk to develop a disease which is due to or accompanied by fatty acid oxidation disorder,
said risk assessment preferably comprising diagnosis, prognosis, prediction and/or monitoring of said disorder.

2. The use according to claim 1, wherein the MCFA is substituted with 3-hydroxy, 3-oxo, and/or wherein the MCFA is unsaturated,
and/or wherein the lipid compound is a free, optionally substituted and/or optionally unsaturated fatty acid with R₁ being -OH,
wherein preferably n = 1 (C8:0-fatty acid, FA 8:0) or n = 2 (C10:0-fatty acid, FA 10:0); or wherein the lipid compound is selected from fatty acyl coenzymes A (CoAs) with R₁ being -SCoA, of formula 2: 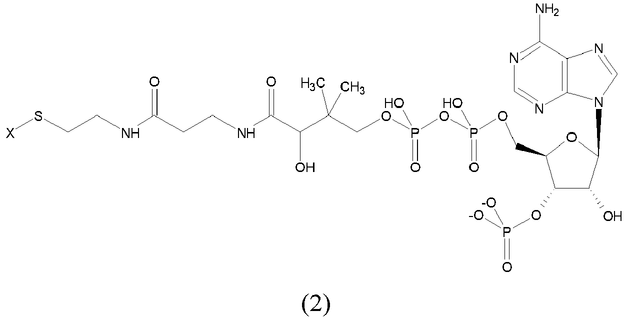
wherein X is a MCFA,
wherein preferably n = 1 (C8:0-acyl-coenzyme A, CoA 8:0) or n = 2 (C10:0-acyl-coenzyme A, CoA 10:0) and wherein said MCFA is optionally substituted and/or unsaturated;
or wherein the lipid compound is selected from acylcarnitines with R₁ being carnitine, of formula 3: 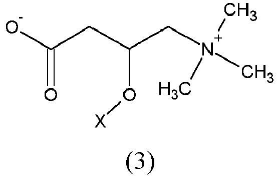
wherein X is a MCFA,
wherein preferably n = 1 (C8:0-acylcarnitine, CAR 8:0) or n = 2 (C10:0-acyl-carnitine, CAR 10:0) and wherein said MCFA is optionally substituted and/or unsaturated;
or wherein the lipid compound is selected from phosphatidylcholines of formula 4: 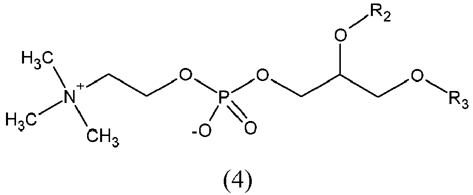 wherein R₂ and R₃ are fatty acyls with at least one of R₂ and R₃ being a MCFA, wherein in said MCFA n is preferably 1 or 2 (C8:0 or C10:0) and wherein said MCFA is optionally substituted and/or unsaturated
more preferably the lipid compound is selected from
PC(8:0/8:0),
PC(10:0/8:0),
PC(8:0/10:0),
PC(10:0/10:0),
PC(16:0/8:0),
PC(8:0/16:0),
PC(16:0/10:0),
PC(10:0/16:0),
PC(10:0/20:4) or
PC(20:4/10:0);
or wherein the lipid compound is selected from compounds of formula 5: 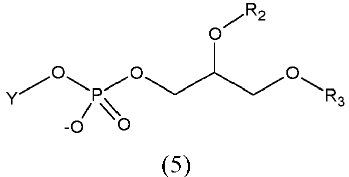 wherein
R₂ and R₃ are H or fatty acyl(s), with at least one of R₂ and R₃ being a MCFA, wherein in said MCFA n is preferably 1 or 2 and wherein said MCFA is optionally substituted and/or unsaturated,
Y is H, or is a head group selected from the group comprising choline, ethanolamine (NH₂-CH₂-CH₂-), inositol, glycerol or serine;
or wherein the lipid compound is selected from compounds of formula 6: 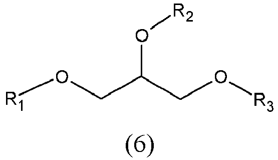 wherein
R₁ is H,
R₂ and R₃ are both fatty acyls with at least one of R₂ and R₃ being a MCFA, wherein in said MCFA n is preferably 1 or 2 and wherein said MCFA is optionally substituted and/or unsaturated,
wherein preferably one of R₂ and R₃ is H and the other one is a MCFA, wherein in said MCFA n is preferably 1 or 2 and wherein said MCFA is optionally substituted and/or unsaturated;
or wherein the lipid compound is selected from compounds of formula 6: 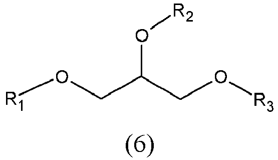 wherein
R₁, R₂ and R₃ are fatty acyls with at least one of R₁, R₂ and R₃ being a MCFA, wherein in said MCFA n is preferably 1 or 2 and wherein said MCFA is optionally substituted and/or unsaturated;
or wherein the lipid compound is selected from compounds of formula 7 or 8 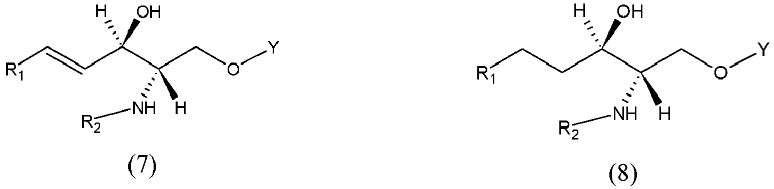 wherein
R₁ is C9-C15 alkyl,
R₂ is a fatty acyl with a MCFA, wherein in said MCFA n is preferably 1 or 2 and wherein said MCFA is optionally substituted and/or unsaturated,
Y is selected from H, hexosyl, dihexosyl, or phosphocholine;
or wherein the lipid compound is selected from compounds of formula 9 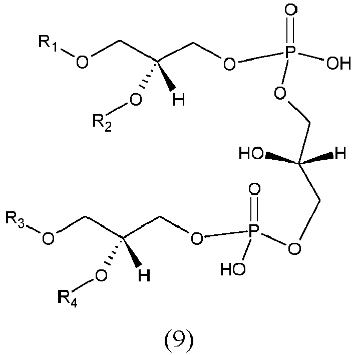 wherein
R₁, R₂, R₃ and R₄ are fatty acyls with at least one of R₁, R₂, R₃ and R₄ being a MCFA, wherein in said MCFA n is preferably 1 or 2 and wherein said MCFA is optionally substituted and/or unsaturated;
or wherein the lipid compound is selected from compounds of formula 10 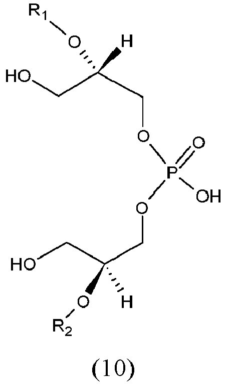 wherein
R₁ and R₂ are fatty acyls with at least one of R₁ and R₂ being a MCFA, wherein in said MCFA n is preferably 1 or 2 and wherein said MCFA is optionally substituted and/or unsaturated.

3. The use according to any of the preceding claims, comprising
determining the relative or absolute concentration of at least one of said lipid compounds in a sample;
or comparing the signal intensities between /determining the molar ratio of at least two lipid compounds in a sample, wherein at least one is a lipid compound as defined in anyone of claims 1 or 2.

4. The use according to any of the claims 1 to 3, wherein the sample is a biological sample from a human,
such as blood, plasma, serum, blood cells (preferably platelets), dried-blood spots, tissue samples from biopsies, cerebrospinal fluid, urine, saliva, or other cells, e.g. neuronal cells, cardiomyocytes;
and/or wherein the disease due to or accompanied by fatty acid oxidation disorder is selected from
cardiovascular disease (CVD),
coronary artery disease (CAD),
stroke,
acute coronary syndrome (ACS),
chronic coronary syndrome (CCS),
heart failure.

5. The use according to any of the preceding claims, wherein determining the relative or absolute concentration of the lipid compound(s) is carried out using mass spectrometry,
wherein, preferably, the mass spectrometry is electrospray ionization-tandem mass spectrometry (QqQ, QTRAP) or electrospray ionization-high-resolution accurate mass spectrometry (QTOF, Orbitrap, FTICR-MS),
and/or wherein, preferably, the mass spectrometry is hyphenated to liquid chromatography and/or ion mobility spectrometry, or both.

6. The use according to any of the preceding claims, wherein at least two of the lipid compounds are used;
and/or wherein the molar ratio of corresponding MCFA- and LCFA-containing lipid compounds, preferably
C8:0-fatty acyl-CoA to C16:0-fatty acyl-CoA,
C8:0-fatty acyl-CoA to C18:0-fatty acyl-CoA
C10:0-fatty acyl-CoA to C16:0-fatty acyl-CoA,
C10:0-fatty acyl-CoA to C18:0-fatty acyl-CoA,
C8:0-fatty acid to C16:0-fatty acid,
C10:0-fatty acid to C16:0-fatty acid,
C8:0-acylcarnitine to C16:0-acylcarnitine,
C10:0-acylcarnitine to C16:0-acylcarnitine,PC(8:0-10:0) to PC(16:0-18:1), PC(10:0-10:0) to PC(16:0-18:1),
PC(16:0-8:0) to PC(16:0-18:1), and/or
PC(20:4-10:0) to PC(16:0-18:1),
is determined;
and/or wherein furthermore the relative or absolute concentration of at least one oxidized phospholipid is determined.

7. The use according to any of the preceding claims, wherein at least one of the following 5 lipid compounds are determined:
PC(8:0/8:0),
PC(10:0-8:0),
PC(10:0/10:0),
PC(10:0-20:4), and
PC(8:0-16:0),
wherein, preferably, the sample are platelets,
and wherein preferably an upregulation of said lipid compounds is indicative for higher risk of disease or higher disease severity;
and wherein optionally furthermore
PE(16:0-18:0), PC(16:0e/16:1) and/or PC(18:1e/16:0) are determined, wherein, preferably, the sample are platelets,
and wherein preferably an upregulation of said lipid compounds is indicative for higher risk of disease or higher disease severity.

8. The use according to claim 7, wherein furthermore at least one lipid selected from a phosphatidylcholine, phosphatidylethanolamine diacylglycerol or triacylglycerol with a docosahexaenoic acid fatty acyl side chain (FA22:6) is determined,
and/or wherein furthermore at least one of
PC(15:0-20:4),
PC(16:0-22:6),
PC(17:0-20:4),
PC(17:0-22:6),
PC(18:0-20:4),
PC(18:0-22:6),
PC(20:0-22:6),
PC(20:4-22:6),
PC(22:5-22:5),
PC(16:1e/20:4),
PC(20:0e/22:6),
LPC 18:0,
LPE 16:0,
LPE 18:0,
PE(18:1e/22:6),
PE(20:4-22:6),
DG(18:0-22:4),
TG(16:0-18:0-22:6),
TG(16:0-20:0-22:6),
TG(18:0-20:4-22:6), and/or
SM(d18: 1/22:0),
is/are determined,
wherein, preferably, the sample are platelets,
and wherein preferably a downregulation is indicative for higher risk of disease or higher disease severity, such as for ACS.

9. Method for assessing the risk to develop a disease which is due to or accompanied by fatty acid oxidation disorder, said method comprising:
(a) providing a sample obtained from a human,
(b) determining the relative or absolute concentration of at least one lipid compound in said sample,
wherein said lipid compound is as defined in claim 1 or 2.

10. The method of claim 9, further comprising
(c) comparing the signal intensities between/determining the molar ratio of at least two lipid compounds in a sample, wherein at least one is a lipid compound as defined in claim 1 or 2.

11. The method of claim 9 or 10, wherein the sample obtained from a human is blood, plasma, serum, blood cells (preferably platelets), dried-blood spots, tissue samples from biopsies, cerebrospinal fluid, urine, saliva, or other cells, e.g. neuronal cells, cardiomyocytes;
and/or wherein the risk assessment comprises diagnosis, prognosis, prediction and/or monitoring of a disease which is due to or accompanied by fatty acid oxidation disorder,
wherein said disease is preferably selected from
cardiovascular disease (CVD),
coronary artery disease (CAD),
stroke,
acute coronary syndrome (ACS),
chronic coronary syndrome (CCS),
heart failure.

12. The method of any one of claims 9 to 11, wherein determining the relative or absolute concentration of the lipid compound(s) is carried out using mass spectrometry,
wherein, preferably, the mass spectrometry is electrospray ionization-tandem mass spectrometry (QqQ, QTRAP) or electrospray ionization-high-resolution accurate mass spectrometry (QTOF, Orbitrap, FTICR-MS),
and/or wherein, preferably, the mass spectrometry is hyphenated to liquid chromatography and/or ion mobility spectrometry, or both.

13. The method of any one of claims 9 to 12, wherein the molar ratio of corresponding MCFA- and LCFA-containing lipid compounds, preferably
C8:0-fatty acyl-CoA to C16:0-fatty acyl-CoA,
C8:0-fatty acyl-CoA to C18:0-fatty acyl-CoA
C10:0-fatty acyl-CoA to C16:0-fatty acyl-CoA,
C10:0-fatty acyl-CoA to C18:0-fatty acyl-CoA,
C8:0-fatty acid to C16:0-fatty acid,
C10:0-fatty acid to C16:0-fatty acid,
C8:0-acylcarnitine to C16:0-acylcarnitine,
C10:0-acylcarnitine to C16:0-acylcarnitine,
PC(8:0-10:0) to PC(16:0-18:1),
PC(10:0-10:0) to PC(16:0-18:1),
PC(16:0-8:0) to PC(16:0-18:1), and/or
PC(20:4-10:0) to PC(16:0-18:1),
is determined;
and/or wherein furthermore the relative or absolute concentration of at least one oxidized phospholipid is determined.

14. The method of any one of claims 9 to 13, wherein at least one of the following 5 lipid compounds is determined:
PC(8:0/8:0),
PC(10:0-8:0),
PC(10:0/10:0),
PC(10:0-20:4), and
PC(8:0-16:0),
wherein, preferably, the sample are platelets,
and wherein preferably an upregulation of said lipid compounds is indicative for higher risk of disease or higher disease severity, such as for ACS;
wherein optionally furthermore
PE(16:0-18:0), PC(16:0e/16:1) and/or PC(18:1e/16:0) are determined, wherein, preferably, the sample are platelets,
and wherein preferably an upregulation of said lipid compounds is indicative for higher risk of disease or higher disease severity, such as for ACS.

15. The method according to claim 14, wherein furthermore at least one lipid selected from a phosphatidylcholine, phosphatidylethanolamine diacylglycerol or triacylglycerol with a docosahexaenoic acid fatty acyl side chain (FA22:6) is determined,
and/or wherein furthermore at least one of
PC(15:0-20:4),
PC(16:0-22:6),
PC(17:0-20:4),
PC(17:0-22:6),
PC(18:0-20:4),
PC(18:0-22:6),
PC(20:0-22:6),
PC(20:4-22:6),
PC(22:5-22:5),
PC(16:1e/20:4),
PC(20:0e/22:6),
LPC 18:0,
LPE 16:0,
LPE 18:0,
PE(18:1e/22:6),
PE(20:4-22:6),
DG(18:0-22:4),
TG(16:0-18:0-22:6),
TG(16:0-20:0-22:6),
TG(18:0-20:4-22:6), and/or
SM(d18: 1/22:0),
is/are determined,
wherein, preferably, the sample are platelets,
and wherein preferably a downregulation is indicative for higher risk of disease or higher disease severity, such as for ACS.

16. The method according to any one of claims 9 to15, comprising the combination with other clinical markers, comprising cardiac troponins, (high-sensitivity, hs) C-reactive protein, ApoB, Lp(a), LDL-C, LDL-P, CETP, Lp-PLA2, sICAM-1, IL-6, IL-18, SAA, MPO, sCD40, oxidized LDL, GPx1 activity, nitrotyrosine, homocysteine, cystatin C, (atrial and B-type) natriuretic peptides (ANP and BNP), ADMA, MMP-9, TIMP-1, fibrinogen, D-dimer, TPA/PAI-1, blood pressure, endothelial dysfunction, arterial stiffness, serum amyloid protein A, TNFalpha, APO-1, urinary and plasma isoprostanes, urinary biopyrrins, plasma malondialdehyde, heart type fatty acid binding protein, myosin light-chain kinase I, myocardial-bound creatine kinase, ST2, collagen propeptides, galectin-3, chromogranin, adiponectin, osteoprotegerin, growth differentiation factor 15, urinary thromboxanes, MCP-1, plasma ceramides.
